# EUROPÄISCHE PATENTSCHRIFT

(11) **EP 1 623 025 B1**
(45) Veröffentlichungstag und Bekanntmachung des Hinweises auf die Patenterteilung: **14.05.2008**
(21) Anmeldenummer: 04729624.9
(22) Anmeldetag: 27.04.2004
(51) Int. Cl.: C12N 9/04, C12Q 1/32, C12Q 1/54, C12N 15/53, C12N 15/63

(54) **GLUKOSE DEHYDROGENASE UND IHRE HERSTELLUNG**
GLUCOSE DEHYDROGENASE AND PRODUCTION THEREOF
GLUCOSE DESHYDROGENASE ET SON PROCEDE DE PRODUCTION

(30) Priorität: 07.05.2003 DE 10320259
(43) Veröffentlichungstag der Anmeldung: 08.02.2006
(73) Patentinhaber: Bayer Technology Services GmbH, 51368 Leverkusen (DE)
(72) Erfinder: MEISSNER, Ruth, 51375 Leverkusen (DE); WEICHEL, Walter, 51519 Odenthal (DE); KOCH, Rainhard, 14532 Kleinmachnow (DE); BACHMATOVA, Irina, 2057 Vilnius (LT); MARCINKEVICIENE, Liucija, 2057 Vilnius (LT); MESKIENE, Rita, 2051 Vilnius (LT); SEMENAITE, Rasa, 2056 Vilnius (LT); CASAITE, Vida, 2050 Vilnius (LT); MESKYS, Rolandas, 2051 Vilnius (LT)
(74) Vertreter: Lütjens, Henning
(86) Internationale Anmeldenummer: PCT/EP2004/004413
(87) Internationale Veröffentlichungsnummer: WO 2004/099399

(56) Entgegenhaltungen:
- EP-A- 1 167 519
- WO-A-02/34919
- PATENT ABSTRACTS OF JAPAN Bd. 1999, Nr. 14, 22. Dezember 1999 (1999-12-22) -& JP 11 243949 A (TOYOBO CO LTD), 14. September 1999 (1999-09-14)
- PATENT ABSTRACTS OF JAPAN Bd. 2000, Nr. 15, 6. April 2001 (2001-04-06) -& JP 2000 354495 A (HAYADE KOJI), 26. Dezember 2000 (2000-12-26)
- LABER B ET AL: "Vitamin B6 biosynthesis: formation of pyridoxine 5'-phosphate from 4-(phosphohydroxy)-l-threonine and 1-deoxy-d-xylulose-5-phosphate by PdxA and PdxJ protein" FEBS LETTERS, ELSEVIER SCIENCE PUBLISHERS, AMSTERDAM, NL, Bd. 449, Nr. 1, 16. April 1999 (1999-04-16), Seiten 45-48, XP004259528 ISSN: 0014-5793
- TAKAHASHI Y ET AL: "CONSTRUCTON AND CHARACTERIZATION OF GLUCOSE ENZYME SENSOR EMPLOYING ENGINEERED WATER SOLUBLE PQQ GLUCOSE DEHYDROGENASE WITH IMPROVED THERMAL STABILITY" PROCEEDINGS OF THE VOICE I/O SYSTEMS APPLICATIONS CONFERENCE, XX, XX, Bd. 68, Nr. 11, 2000, Seiten 907-911, XP009028017
- SODE K ET AL: "INCREASING THE THERMAL STABILITY OF THE WATER-SOLUBLE PYRROLOQUINOLINE QUINONE GLUCOSE DEHYDROGENASE BY SINGLE AMINO ACID REPLACEMENT" ENZYME AND MICROBIAL TECHNOLOGY, STONEHAM, MA, US, Bd. 26, Nr. 7, April 2001 (2001-04), Seiten 491-496, XP001155036 ISSN: 0141-0229
- PATENT ABSTRACTS OF JAPAN Bd. 2000, Nr. 19, 5. Juni 2001 (2001-06-05) & JP 2001 037483 A (HAYADE KOJI), 13. Februar 2001 (2001-02-13) -& JP 2001 037483 A (HAYADE KOJI) 13. Februar 2001 (2001-02-13)
- PATENT ABSTRACTS OF JAPAN Bd. 2000, Nr. 24, 11. Mai 2001 (2001-05-11) -& JP 2001 197888 A (HAYADE KOJI), 24. Juli 2001 (2001-07-24)
- CLETON-JANSEN A-M ET AL: "CLONING, CHARACTERIZATION AND DNA SEQUENCING OF THE GENE ENCODING THE MR 50000 QUINOPROTEIN GLUCOSE DEHYDROGENASE FROM ACINETOBACTER CALCOACETICUS" MOLECULAR AND GENERAL GENETICS, SPRINGER VERLAG, BERLIN, DE, Bd. 217, Nr. 2/3, Juni 1989 (1989-06), Seiten 430-436, XP000990490 ISSN: 0026-8925

### SEQUENCE LISTING

<110> Bayer AG
<120> Neuartige Glukose Dehydrogenase und ihre Herstellung
<130> LeA36627
<160> 28
<170> PatentIn Version 3.1
<210> 1
   <211> 1437
   <212> DNA
   <213> Acinetobacter sp.
<400> 1
   atgaataaac atttattggc taaaattgct ttattaagcg ctgttcagct agttacactc     60
   tcagcatttg ctgatgttcc tctaactcca tctcaatttg ctaaagcgaa atcagagaac    120
   tttgacaaga aagttattct atctaatcta aataagccgc atgctttgtt atggggacca    180
   gataatcaaa tttggttaac tgagcgagca acaggtaaga ttctaagagt taatccagag    240
   tcgggtagtg taaaaacagt ttttcaggta ccagagattg tcaatgatgc tgatgggcag     300
   aatggtttat taggttttgc cttccatcct gattttaaaa ataatcctta tatctatatt    360
   tcaggtacat ttaaaaatcc gaaatctaca gataaagaat taccgaacca aacgattatt    420
   cgtcgttata cctataataa atcaacagat acgctcgaga agccagtcga tttattagca    480
   ggattacctt catcaaaaga ccatcagtca ggtcgtcttg tcattgggcc agatcaaaag    540
   atttattata cgattggtga ccaagggcgt aaccagcttg cttatttgtt cttgccaaat    600
   caagcacaac atacgccaac tcaacaagaa ctgaatggta aagactatca cacctatatg    660
   ggtaaagtac tacgcttaaa tcttgatgga agtattccaa aggataatcc aagttttaac    720
   ggggtggtta gccatattta tacacttgga catcgtaatc cgcagggctt agcattcact    780
   ccaaatggta aattattgca gtctgaacaa ggcccaaact ctgacgatga aattaacctc    840
   attgtcaaag gtggcaatta tggttggccg aatgtagcag gttataaaga tgatagtggc    900
   tatgcttatg caaattattc agcagcagcc aataagtcaa ttaaggattt agctcaaaat    960
   ggagtaaaag tagccgcagg ggtccctgtg acgaaagaat ctgaatggac tggtaaaaac   1020
   tttgtcccac cattaaaaac tttatatacc gttcaagata cctacaacta taacgatcca   1080
   acttgtggag agatgaccta catttgctgg ccaacagttg caccgtcatc tgcctatgtc   1140
   tataagggcg gtaaaaaagc aattactggt tgggaaaata cattattggt tccatcttta   1200
   aaacgtggtg tcattttccg tattaagtta gatccaactt atagcactac ttatgatgac   1260
   gctgtaccga tgtttaagag caacaaccgt tatcgtgatg tgattgcaag tccagatggg   1320
   aatgtcttat atgtattaac tgatactgcc ggaaatgtcc aaaaagatga tggctcagta   1380
   acaaatacat tagaaaaccc aggatctctc attaagttca cctataaggc taagtaa      1437
<210> 2
   <211> 478
   <212> PRT
   <213> Acinetobacter sp.
<400> 2
   Met Asn Lys His Leu Leu Ala Lys Ile Ala Leu Leu Ser Ala Val Gln
   1               5                   10                  15
   Leu Val Thr Leu Ser Ala Phe Ala Asp Val Pro Leu Thr Pro Ser Gln
               20                  25                  30
   Phe Ala Lys Ala Lys Ser Glu Asn Phe Asp Lys Lys Val Ile Leu Ser
           35                  40                  45
   Asn Leu Asn Lys Pro His Ala Leu Leu Trp Gly Pro Asp Asn Gln Ile
       50                  55                  60
   Trp Leu Thr Glu Arg Ala Thr Gly Lys Ile Leu Arg Val Asn Pro Glu
   65                  70                  75                  80
   Ser Gly Ser Val Lys Thr Val Phe Gln Val Pro Glu Ile Val Asn Asp
                   85                  90                  95
   Ala Asp Gly Gln Asn Gly Leu Leu Gly Phe Ala Phe His Pro Asp Phe
               100                 105                 110
   Lys Asn Asn Pro Tyr Ile Tyr Ile Ser Gly Thr Phe Lys Asn Pro Lys
   115 120 125
   Ser Thr Asp Lys Glu Leu Pro Asn Gln Thr Ile Ile Arg Arg Tyr Thr
   130 135 140
   Tyr Asn Lys Ser Thr Asp Thr Leu Glu Lys Pro Val Asp Leu Leu Ala
   145 150 155 160
   Gly Leu Pro Ser Ser Lys Asp His Gln Ser Gly Arg Leu Val Ile Gly
   165 170 175
   Pro Asp Gln Lys Ile Tyr Tyr Thr Ile Gly Asp Gln Gly Arg Asn Gln
   180 185 190
   Leu Ala Tyr Leu Phe Leu Pro Asn Gln Ala Gln His Thr Pro Thr Gln
   195 200 205
   Gln Glu Leu Asn Gly Lys Asp Tyr His Thr Tyr Met Gly Lys Val Leu
   210 215 220
   Arg Leu Asn Leu Asp Gly Ser Ile Pro Lys Asp Asn Pro Ser Phe Asn
   225 230 235 240
   Gly Val Val Ser His Ile Tyr Thr Leu Gly His Arg Asn Pro Gln Gly
   245 250 255
   Leu Ala Phe Thr Pro Asn Gly Lys Leu Leu Gln Ser Glu Gln Gly Pro
   260 265 270
   Asn Ser Asp Asp Glu Ile Asn Leu Ile Val Lys Gly Gly Asn Tyr Gly
   275 280 285
   Trp Pro Asn Val Ala Gly Tyr Lys Asp Asp Ser Gly Tyr Ala Tyr Ala
   290 295 300
   Asn Tyr Ser Ala Ala Ala Asn Lys Ser Ile Lys Asp Leu Ala Gln Asn
   305 310 315 320
   Gly Val Lys Val Ala Ala Gly Val Pro Val Thr Lys Glu Ser Glu Trp
   325 330 335
   Thr Gly Lys Asn Phe Val Pro Pro Leu Lys Thr Leu Tyr Thr Val Gln
   340 345 350
   Asp Thr Tyr Asn Tyr Asn Asp Pro Thr Cys Gly Glu Met Thr Tyr Ile
   355 360 365
   Cys Trp Pro Thr Val Ala Pro Ser Ser Ala Tyr Val Tyr Lys Gly Gly
       370                 375                 380
   Lys Lys Ala Ile Thr Gly Trp Glu Asn Thr Leu Leu Val Pro Ser Leu
   385                 390                 395                 400
   Lys Arg Gly Val Ile Phe Arg Ile Lys Leu Asp Pro Thr Tyr Ser Thr
                   405                 410                 415
   Thr Tyr Asp Asp Ala Val Pro Met Phe Lys Ser Asn Asn Arg Tyr Arg
               420                 425                 430
   Asp Val Ile Ala Ser Pro Asp Gly Asn Val Leu Tyr Val Leu Thr Asp
           435                 440                 445
   Thr Ala Gly Asn Val Gln Lys Asp Asp Gly Ser Val Thr Asn Thr Leu
       450                 455                 460
   Glu Asn Pro Gly Ser Leu Ile Lys Phe Thr Tyr Lys Ala Lys
   465                 470                 475
<210> 3
   <211> 1443
   <212> DNA
   <213> Acinetobacter sp.
<400> 3
   atgaataaac atttattagc aaaaatcact cttttaggtg ctgcacaact atttacgttt      60
   catacggcat ttgcagatat acctctgaca cctgctcagt tcgcaaaagc gaaaacagaa     120
   aattttgata aaaaagtgat tctgtccaat ttaaataaac cacatgcttt gttatggggg     180
   ccagataatc aaatttggtt aaccgaacgt gcaactggca aaattttaag agtaaatcct     240
   gtatctggta gcgcgaaaac agtatttcag gttcctgaaa ttgtgagtga tgctgatggg     300
   caaaatggtt tgttaggttt tgcttttcat cctgacttta aacataaccc ttatatctat     360
   atttcaggca cttttaaaaa tccaaaatct acagataaag agttacctaa tcagacaatt     420
   attcgtagat atacctataa taaaactaca gatacatttg aaaagcctat tgatttgatt     480
   gcaggtttac cgtcatcaaa agatcatcag tctggtcgtc tcgttattgg tccagaccaa     540
   aaaatctact atacgattgg tgaccaaggt cgtaatcagt tagcttatct attcttatcg     600
   aatcaggcac agcatactcc gactcagcaa gagctcaata gtaaagacta ccatacatat     660
   atgggtaaag tattacgctt aaatctggac ggcagtatac ctaaagacaa cccaagcttt    720
   aacggcgtag tgagtcatat ctacacttta gggcaccgta atccacaagg tttagcattt    780
   gccccaaatg gaaagctttt acaatctgag caagggccaa attctgatga tgaaattaac    840
   cttgtattaa aaggtggtaa ctatggctgg ccaaatgtag ctggttataa agatgacagt    900
   ggttatgcct atgcaaacta ttcggcagca accaataaat cacaaattaa agatttagct    960
   caaaacggga taaaagtagc aacaggtgtt cctgtgacta aagagtctga atggactggt   1020
   aaaaactttg tgccaccttt gaaaacttta tatacggtac aagataccta taactataat   1080
   gaccctactt gtggtgagat ggcatatatt tgctggccaa cggttgcacc gtcatcggca   1140
   tatgtatata cgggaggcaa aaaagcgatt ccagggtggg aaaatacatt attggtccca   1200
   tctttaaaac gtggggtgat tttccgtatt aaattggacc cgacatatag cacgactttg   1260
   gatgatgcta tcccaatgtt taaaagcaat aaccgttatc gtgatgtcat cgctagtcca   1320
   gaaggtaata ccttatatgt gctgactgat acagcgggaa atgtacaaaa agatgatggt   1380
   tcagtcactc atactttaga gaatcccggt tctctcatta aatttacata taacggtaag   1440
   taa                                                                 1443
<210> 4
   <211> 480
   <212> PRT
   <213> Acinetobacter sp.
<400> 4
   Met Asn Lys His Leu Leu Ala Lys Ile Thr Leu Leu Gly Ala Ala Gln
   1               5                   10                  15
   Leu Phe Thr Phe His Thr Ala Phe Ala Asp Ile Pro Leu Thr Pro Ala
               20                  25                  30
   Gln Phe Ala Lys Ala Lys Thr Glu Asn Phe Asp Lys Lys Val Ile Leu
           35                  40                  45
   Ser Asn Leu Asn Lys Pro His Ala Leu Leu Trp Gly Pro Asp Asn Gln
       50                  55                  60
   Ile Trp Leu Thr Glu Arg Ala Thr Gly Lys Ile Leu Arg Val Asn Pro
   65                  70                  75                  80
   Val Ser Gly Ser Ala Lys Thr Val Phe Gln Val Pro Glu Ile Val Ser
   85 90 95
   Asp Ala Asp Gly Gln Asn Gly Leu Leu Gly Phe Ala Phe His Pro Asp
   100 105 110
   Phe Lys His Asn Pro Tyr Ile Tyr Ile Ser Gly Thr Phe Lys Asn Pro
   115 120 125
   Lys Ser Thr Asp Lys Glu Leu Pro Asn Gln Thr Ile Ile Arg Arg Tyr
   130 135 140
   Thr Tyr Asn Lys Thr Thr Asp Thr Phe Glu Lys Pro Ile Asp Leu Ile
   145 150 155 160
   Ala Gly Leu Pro Ser Ser Lys Asp His Gln Ser Gly Arg Leu Val Ile
   165 170 175
   Gly Pro Asp Gln Lys Ile Tyr Tyr Thr Ile Gly Asp Gln Gly Arg Asn
   180 185 190
   Gln Leu Ala Tyr Leu Phe Leu Ser Asn Gln Ala Gln His Thr Pro Thr
   195 200 205
   Gln Gln Glu Leu Asn Ser Lys Asp Tyr His Thr Tyr Met Gly Lys Val
   210 215 220
   Leu Arg Leu Asn Leu Asp Gly Ser Ile Pro Lys Asp Asn Pro Ser Phe
   225 230 235 240
   Asn Gly Val Val Ser His Ile Tyr Thr Leu Gly His Arg Asn Pro Gln
   245 250 255
   Gly Leu Ala Phe Ala Pro Asn Gly Lys Leu Leu Gln Ser Glu Gln Gly
   260 265 270
   Pro Asn Ser Asp Asp Glu Ile Asn Leu Val Leu Lys Gly Gly Asn Tyr
   275 280 285
   Gly Trp Pro Asn Val Ala Gly Tyr Lys Asp Asp Ser Gly Tyr Ala Tyr
   290 295 300
   Ala Asn Tyr Ser Ala Ala Thr Asn Lys Ser Gln Ile Lys Asp Leu Ala
   305 310 315 320
   Gln Asn Gly Ile Lys Val Ala Thr Gly Val Pro Val Thr Lys Glu Ser
   325 330 335
   Glu Trp Thr Gly Lys Asn Phe Val Pro Pro Leu Lys Thr Leu Tyr Thr
   340 345 350
   Val Gln Asp Thr Tyr Asn Tyr Asn Asp Pro Thr Cys Gly Glu Met Ala
   355 360 365
   Tyr Ile Cys Trp Pro Thr Val Ala Pro Ser Ser Ala Tyr Val Tyr Thr
   370 375 380
   Gly Gly Lys Lys Ala Ile Pro Gly Trp Glu Asn Thr Leu Leu Val Pro
   385 390 395 400
   Ser Leu Lys Arg Gly Val Ile Phe Arg Ile Lys Leu Asp Pro Thr Tyr
   405 410 415
   Ser Thr Thr Leu Asp Asp Ala Ile Pro Met Phe Lys Ser Asn Asn Arg
   420 425 430
   Tyr Arg Asp Val Ile Ala Ser Pro Glu Gly Asn Thr Leu Tyr Val Leu
   435 440 445
   Thr Asp Thr Ala Gly Asn Val Gln Lys Asp Asp Gly Ser Val Thr His
   450 455 460
   Thr Leu Glu Asn Pro Gly Ser Leu Ile Lys Phe Thr Tyr Asn Gly Lys
   465 470 475 480
<210> 5
   <211> 1443
   <212> DNA
   <213> Acinetobacter sp.
<400> 5
   atgaataaac atttattggc taaaattact ttattaggtg ctgctcagct acttacgctc 60
   aattcagcat ttgctgatgt ccctcttact ccatctcaat ttgctaaagc gaaaacagaa 120
   agctttgata agaaagttct tctatctaat ttaaataagc cacatgcttt gttgtggggg 180
   cctgataatc aaatttggtt aacggagcgg gcaacaggta agattctaag agtgaatcca 240
   gagtcgggca gtgtgaaaac agtttttcag gttcctgaga ttgtaaatga tgctgatgga 300
   caaaacggtt tattaggttt tgcttttcat cctgacttta aacataatcc ttatatctat 360
   gtttcaggta catttaaaaa tccgaaatct acagataaag aattaccgaa tcaaactatt 420
   attcgtcgat atacctataa caaagcaaca gatactcttg agaaaccagt agatttatta    480
   gcaggattac cttcatcgaa agaccatcag tcgggtcgcc ttgttattgg tccagaccaa    540
   aagatttact atacgattgg tgatcaggga cgtaaccagc tggcttattt attcttacca    600
   aatcaagcac agcatacgcc gactcaacag gaactgagcg gcaaagacta tcatacttat    660
   atgggtaaag tattacgctt aaatctggat ggaagtattc caaaagataa tccaagcttt    720
   aacggtgtaa ttagccatat ttatacgctc ggtcatcgta acccacaggg cttggcattt    780
   actccaaatg gtaaactgtt gcaatctgaa caaggtccaa actctgatga tgaaattaat    840
   ctcattgtta aaggtggtaa ctatggctgg ccaaatgtag ctggttataa agatgacagt    900
   ggttatgcct atgcaaatta ttcggcagca accaataagt cacaaattaa agatttaggg    960
   caaaatggag taaaagtagc ggcaggtgta cctgtgatga aagagtctga atggagtggt   1020
   aaaaactttg taccgccgtt aaaaacttta tataccgtcc aagataccta taactataat   1080
   gacccaactt gtggggatat gacctacatt tgctggccaa cagttgcgcc atcatctgct   1140
   tatgtctata aggggggcaa aaaagcaatt tctggttggg aaaatacatt attggttcca   1200
   tctttaaagc gtggtgttat tttccgtatt aagttagatc caacttacag tgctacttat   1260
   gatgatgctg taccgatgtt taagagcaat aaccgttatc gtgacgtgat tgcaagtcca   1320
   gatgggaatg ttttatatgt attgactgat acttccggaa atgtccaaaa ggatgatggc   1380
   tctgtaacga atacattaga aaatccagga tctctgatta gatttacata taaagctcag   1440
   taa                                                                 1443
<210> 6
   <211> 480
   <212> PRT
   <213> Acinetobacter sp.
<400> 6
   Met Asn Lys His Leu Leu Ala Lys Ile Thr Leu Leu Gly Ala Ala Gln
   1               5                   10                  15
   Leu Leu Thr Leu Asn Ser Ala Phe Ala Asp Val Pro Leu Thr Pro Ser
               20                  25                  30
   Gln Phe Ala Lys Ala Lys Thr Glu Ser Phe Asp Lys Lys Val Leu Leu
           35                  40                  45
   Ser Asn Leu Asn Lys Pro His Ala Leu Leu Trp Gly Pro Asp Asn Gln
   50 55 60
   Ile Trp Leu Thr Glu Arg Ala Thr Gly Lys Ile Leu Arg Val Asn Pro
   65 70 75 80
   Glu Ser Gly Ser Val Lys Thr Val Phe Gln Val Pro Glu Ile Val Asn
   85 90 95
   Asp Ala Asp Gly Gln Asn Gly Leu Leu Gly Phe Ala Phe His Pro Asp
   100 105 110
   Phe Lys His Asn Pro Tyr Ile Tyr Val Ser Gly Thr Phe Lys Asn Pro
   115 120 125
   Lys Ser Thr Asp Lys Glu Leu Pro Asn Gln Thr Ile Ile Arg Arg Tyr
   130 135 140
   Thr Tyr Asn Lys Ala Thr Asp Thr Leu Glu Lys Pro Val Asp Leu Leu
   145 150 155 160
   Ala Gly Leu Pro Ser Ser Lys Asp His Gln Ser Gly Arg Leu Val Ile
   165 170 175
   Gly Pro Asp Gln Lys Ile Tyr Tyr Thr Ile Gly Asp Gln Gly Arg Asn
   180 185 190
   Gln Leu Ala Tyr Leu Phe Leu Pro Asn Gln Ala Gln His Thr Pro Thr
   195 200 205
   Gln Gln Glu Leu Ser Gly Lys Asp Tyr His Thr Tyr Met Gly Lys Val
   210 215 220
   Leu Arg Leu Asn Leu Asp Gly Ser Ile Pro Lys Asp Asn Pro Ser Phe
   225 230 235 240
   Asn Gly Val Ile Ser His Ile Tyr Thr Leu Gly His Arg Asn Pro Gln
   245 250 255
   Gly Leu Ala Phe Thr Pro Asn Gly Lys Leu Leu Gln Ser Glu Gln Gly
   260 265 270
   Pro Asn Ser Asp Asp Glu Ile Asn Leu Ile Val Lys Gly Gly Asn Tyr
   275 280 285
   Gly Trp Pro Asn Val Ala Gly Tyr Lys Asp Asp Ser Gly Tyr Ala Tyr
   290 295 300
   Ala Asn Tyr Ser Ala Ala Thr Asn Lys Ser Gln Ile Lys Asp Leu Gly
   305 310 315 320
   Gln Asn Gly Val Lys Val Ala Ala Gly Val Pro Val Met Lys Glu Ser
   325 330 335
   Glu Trp Ser Gly Lys Asn Phe Val Pro Pro Leu Lys Thr Leu Tyr Thr
   340 345 350
   Val Gln Asp Thr Tyr Asn Tyr Asn Asp Pro Thr Cys Gly Asp Met Thr
   355 360 365
   Tyr Ile Cys Trp Pro Thr Val Ala Pro Ser Ser Ala Tyr Val Tyr Lys
   370 375 380
   Gly Gly Lys Lys Ala Ile Ser Gly Trp Glu Asn Thr Leu Leu Val Pro
   385 390 395 400
   Ser Leu Lys Arg Gly Val Ile Phe Arg Ile Lys Leu Asp Pro Thr Tyr
   405 410 415
   Ser Ala Thr Tyr Asp Asp Ala Val Pro Met Phe Lys Ser Asn Asn Arg
   420 425 430
   Tyr Arg Asp Val Ile Ala Ser Pro Asp Gly Asn Val Leu Tyr Val Leu
   435 440 445
   Thr Asp Thr Ser Gly Asn Val Gln Lys Asp Asp Gly Ser Val Thr Asn
   450 455 460
   Thr Leu Glu Asn Pro Gly Ser Leu Ile Arg Phe Thr Tyr Lys Ala Gln
   465 470 475 480
<210> 7
   <211> 1443
   <212> DNA
   <213> Acinetobacter sp.
<400> 7
   atgaataaac atttattggc taaaattact ttattaggtg ctgctcagct acttacgctc 60
   aattcagcat ttgctgatgt ccctcttaca ccatctcaat ttgctaaagc gaaaacagaa 120
   agctttgata agaaagttct tctatctaat ttaaataagc cacatgcttt gttgtgggga 180
   cctgataatc aaatttggtt aacggagcgg gcaacaggta agattctaag agttaatcca 240
   gagtcgggca gtgtaaaaac agtttttcag gttcctgaga ttgtaaatga tgctgatgga 300
   caaaacggtt tattgggttt tgcctttcat cctgacttta aaaataatcc ttatatctat 360
   gtttcaggta catttaaaaa tccgaaatct acagataaag aattaccgaa tcaaactatt 420
   atccgtcgat atacctataa caaggcaaca gatacccttg agaaaccagt agatttattg 480
   gcaggattac cttcatcgaa agaccatcag tcgggtcgtc ttgtgattgg tccagaccaa 540
   aagatttact atacgattgg tgatcaggga cgtaaccagc tggcttattt attcttacca 600
   aatcaagcac agcatacgcc gactcaacag gaactgagcg gcaaagacta tcatacctat 660
   atgggtaaag tattgcgctt aaatctggat ggaagtattc caaaagataa tccaagcttt 720
   aacggtgtaa ttagccatat ttatacgctc ggtcatcgta acccacaggg cttggcattt 780
   actccaaatg gtaaactgtt gcaatctgaa cagggtccaa actctgatga tgaaattaac 840
   ctcattgtca aaggtggtaa ctatggctgg ccaaatgtag cgggttataa agatgatagt 900
   ggttatgcct atgcaaatta ttcggcagca agcaataaag cacaaattaa agatttagga 960
   caaaatggtt taaaagtggc ggcaggtgta cctgtgatga aagagtctga atggactggt 1020
   aaaaactttg taccgccgtt aaaaacttta tataccgtcc aagataccta taactataat 1080
   gacccaactt gtggggatat gacctacatt tgctggccaa cggttgcgcc gtcatctgct 1140
   tatgtctata agggaggcaa aaaagcaatt tctggttggg aaaatacatt attggttcca 1200
   tctttaaagc gcggtgttat tttccgtatt aagctagatc caacttacag tactacttat 1260
   gatgatgctg tgccgatgtt taagagcaac aatcgttatc gtgacgtgat tgcaagtcca 1320
   gatgggaatg ttttatatgt attgactgat acttccggaa atgtccaaaa agatgatggt 1380
   tctgtaacga atacattaga aaacccagga tctctgatta gatttacata taaagctcag 1440
   taa 1443
<210> 8
   <211> 480
   <212> PRT
   <213> Acinetobacter sp.
<400> 8
   Met Asn Lys His Leu Leu Ala Lys Ile Thr Leu Leu Gly Ala Ala Gln
   1 5 10 15
   Leu Leu Thr Leu Asn Ser Ala Phe Ala Asp Val Pro Leu Thr Pro Ser
   20 25 30
   Gln Phe Ala Lys Ala Lys Thr Glu Ser Phe Asp Lys Lys Val Leu Leu
   35 40 45
   Ser Asn Leu Asn Lys Pro His Ala Leu Leu Trp Gly Pro Asp Asn Gln
   50 55 60
   Ile Trp Leu Thr Glu Arg Ala Thr Gly Lys Ile Leu Arg Val Asn Pro
   65 70 75 80
   Glu Ser Gly Ser Val Lys Thr Val Phe Gln Val Pro Glu Ile Val Asn
   85 90 95
   Asp Ala Asp Gly Gln Asn Gly Leu Leu Gly Phe Ala Phe His Pro Asp
   100 105 110
   Phe Lys Asn Asn Pro Tyr Ile Tyr Val Ser Gly Thr Phe Lys Asn Pro
   115 120 125
   Lys Ser Thr Asp Lys Glu Leu Pro Asn Gln Thr Ile Ile Arg Arg Tyr
   130 135 140
   Thr Tyr Asn Lys Ala Thr Asp Thr Leu Glu Lys Pro Val Asp Leu Leu
   145 150 155 160
   Ala Gly Leu Pro Ser Ser Lys Asp His Gln Ser Gly Arg Leu Val Ile
   165 170 175
   Gly Pro Asp Gln Lys Ile Tyr Tyr Thr Ile Gly Asp Gln Gly Arg Asn
   180 185 190
   Gln Leu Ala Tyr Leu Phe Leu Pro Asn Gln Ala Gln His Thr Pro Thr
   195 200 205
   Gln Gln Glu Leu Ser Gly Lys Asp Tyr His Thr Tyr Met Gly Lys Val
   210 215 220
   Leu Arg Leu Asn Leu Asp Gly Ser Ile Pro Lys Asp Asn Pro Ser Phe
   225 230 235 240
   Asn Gly Val Ile Ser His Ile Tyr Thr Leu Gly His Arg Asn Pro Gln
   245 250 255
   Gly Leu Ala Phe Thr Pro Asn Gly Lys Leu Leu Gln Ser Glu Gln Gly
   260 265 270
   Pro Asn Ser Asp Asp Glu Ile Asn Leu Ile Val Lys Gly Gly Asn Tyr
   275 280 285
   Gly Trp Pro Asn Val Ala Gly Tyr Lys Asp Asp Ser Gly Tyr Ala Tyr
   290 295 300
   Ala Asn Tyr Ser Ala Ala Ser Asn Lys Ala Gln Ile Lys Asp Leu Gly
   305 310 315 320
   Gln Asn Gly Leu Lys Val Ala Ala Gly Val Pro Val Met Lys Glu Ser
   325 330 335
   Glu Trp Thr Gly Lys Asn Phe Val Pro Pro Leu Lys Thr Leu Tyr Thr
   340 345 350
   Val Gln Asp Thr Tyr Asn Tyr Asn Asp Pro Thr Cys Gly Asp Met Thr
   355 360 365
   Tyr Ile Cys Trp Pro Thr Val Ala Pro Ser Ser Ala Tyr Val Tyr Lys
   370 375 380
   Gly Gly Lys Lys Ala Ile Ser Gly Trp Glu Asn Thr Leu Leu Val Pro
   385 390 395 400
   Ser Leu Lys Arg Gly Val Ile Phe Arg Ile Lys Leu Asp Pro Thr Tyr
   405 410 415
   Ser Thr Thr Tyr Asp Asp Ala Val Pro Met Phe Lys Ser Asn Asn Arg
   420 425 430
   Tyr Arg Asp Val Ile Ala Ser Pro Asp Gly Asn Val Leu Tyr Val Leu
   435 440 445
   Thr Asp Thr Ser Gly Asn Val Gln Lys Asp Asp Gly Ser Val Thr Asn
   450 455 460
   Thr Leu Glu Asn Pro Gly Ser Leu Ile Arg Phe Thr Tyr Lys Ala Gln
   465 470 475 480
<210> 9
   <211> 1443
   <212> DNA
   **<213>** Acinetobacter sp.
<400> 9
   atgaataaac atttattggc taaaattact ttattaggtg ctgctcagct acttacactc    60
   aattcagcat ttgctgatgt ccctcttact ccatctcaat ttgctaaagc gaaaacagaa   120
   agctttgata agaaagttct tctatctaat ttaaataagc cgcatgcttt gttgtgggga   180
   cctgataatc aaatttggtt aacagagcgg gcaacaggta agattctaag agttaaccct   240
   gaatcaggca gtgtaaaaac agtttttcag gttcctgaga ttgtaaatga tgctgatgga   300
   caaaacgggt tattgggttt tgcctttcat cctgacttta aaaataatcc ttatatctat   360
   gtttcaggta catttaaaaa tccgaaatct acagataaag aattaccgaa tcaaactatt   420
   atccgtcgat atacctataa caaagcaaca gatactcttg agaaaccagt agatttatta   480
   gcaggattac cttcatcgaa agaccatcag tcgggtcgcc ttgtgattgg tccagaccaa   540
   aaaatttact atacgattgg tgatcagggg cgtaaccagc ttgcttattt attcttacca   600
   aatcaggcac aacatacgcc gactcaacag gaactgagcg gcaaagacta tcatacctat   660
   atgggtaaag tattacgctt aaatctggat ggaagtattc caaaagataa tccaagcttt   720
   aacggtgtaa ttagccatat ttatacgctc ggtcatcgta acccacaggg cttggcattt   780
   actccaaatg gtaaactgtt gcaatctgaa cagggtccaa actctgatga tgaaattaac   840
   ctcattgtta aaggtggtaa ctatggctgg ccaaatgcgg cgggttataa agatgacagt   900
   ggttatgcct atgcaaatta ttcggcagca accaataagt cacaaattaa agatttaggg   960
   caaaatggag taaaagtagc agctggcgtt ccagtgacta aagagtctga atggactggt  1020
   aaaaactttg taccgccgtt aaaaacttta tataccgtcc aagataccta taactataat  1080
   gacccaacct gtggggagat gacctacatt tgctggccaa cagttgcgcc atcatctgct  1140
   tatgtctata agggaggcaa aaaagcaatt tctggttggg aaaatacctt attggttcca  1200
   tctttaaagc gtggtgttat ttttcgtatt aagctagatc caacttacag tgctacttat  1260
   gatgatgctg tgccgatgtt taagagcaac aatcgttatc gtgacgtgat tgcaagtcca  1320
   gatggaaatg ttttatatgt attgactgat acttccggaa atgtccaaaa agatgatggt  1380
   tctgtaacga atacattaga aaacccagga tctctgatta gatttacata taaagctcag  1440
   taa                                                                1443
<210> 10
   <211> 480
   <212> PRT
   <213> Acinetobacter sp.
<400> 10
   Met Asn Lys His Leu Leu Ala Lys Ile Thr Leu Leu Gly Ala Ala Gln
   1               5                   10                  15
   Leu Leu Thr Leu Asn Ser Ala Phe Ala Asp Val Pro Leu Thr Pro Ser
               20                  25                  30
   Gln Phe Ala Lys Ala Lys Thr Glu Ser Phe Asp Lys Lys Val Leu Leu
           35                  40                  45
   Ser Asn Leu Asn Lys Pro His Ala Leu Leu Trp Gly Pro Asp Asn Gln
       50                  55                  60
   Ile Trp Leu Thr Glu Arg Ala Thr Gly Lys Ile Leu Arg Val Asn Pro
   65                  70                  75                  80
   Glu Ser Gly Ser Val Lys Thr Val Phe Gln Val Pro Glu Ile Val Asn
                   85                  90                  95
   Asp Ala Asp Gly Gln Asn Gly Leu Leu Gly Phe Ala Phe His Pro Asp
               100                 105                 110
   Phe Lys Asn Asn Pro Tyr Ile Tyr Val Ser Gly Thr Phe Lys Asn Pro
           115                 120                 125
   Lys Ser Thr Asp Lys Glu Leu Pro Asn Gln Thr Ile Ile Arg Arg Tyr
       130                 135                 140
   Thr Tyr Asn Lys Ala Thr Asp Thr Leu Glu Lys Pro Val Asp Leu Leu
   145                 150                 155                 160
   Ala Gly Leu Pro Ser Ser Lys Asp His Gln Ser Gly Arg Leu Val Ile
                   165                 170                 175
   Gly Pro Asp Gln Lys Ile Tyr Tyr Thr Ile Gly Asp Gln Gly Arg Asn
               180                 185                 190
   Gln Leu Ala Tyr Leu Phe Leu Pro Asn Gln Ala Gln His Thr Pro Thr
           195                 200                 205
   Gln Gln Glu Leu Ser Gly Lys Asp Tyr His Thr Tyr Met Gly Lys Val
       210                 215                 220
   Leu Arg Leu Asn Leu Asp Gly Ser Ile Pro Lys Asp Asn Pro Ser Phe
   225                 230                 235                 240
   Asn Gly Val Ile Ser His Ile Tyr Thr Leu Gly His Arg Asn Pro Gln
   245 250 255
   Gly Leu Ala Phe Thr Pro Asn Gly Lys Leu Leu Gln Ser Glu Gln Gly
   260 265 270
   Pro Asn Ser Asp Asp Glu Ile Asn Leu Ile Val Lys Gly Gly Asn Tyr
   275 280 285
   Gly Trp Pro Asn Ala Ala Gly Tyr Lys Asp Asp Ser Gly Tyr Ala Tyr
   290 295 300
   Ala Asn Tyr Ser Ala Ala Thr Asn Lys Ser Gln Ile Lys Asp Leu Gly
   305 310 315 320
   Gln Asn Gly Val Lys Val Ala Ala Gly Val Pro Val Thr Lys Glu Ser
   325 330 335
   Glu Trp Thr Gly Lys Asn Phe Val Pro Pro Leu Lys Thr Leu Tyr Thr
   340 345 350
   Val Gln Asp Thr Tyr Asn Tyr Asn Asp Pro Thr Cys Gly Glu Met Thr
   355 360 365
   Tyr Ile Cys Trp Pro Thr Val Ala Pro Ser Ser Ala Tyr Val Tyr Lys
   370 375 380
   Gly Gly Lys Lys Ala Ile Ser Gly Trp Glu Asn Thr Leu Leu Val Pro
   385 390 395 400
   Ser Leu Lys Arg Gly Val Ile Phe Arg Ile Lys Leu Asp Pro Thr Tyr
   405 410 415
   Ser Ala Thr Tyr Asp Asp Ala Val Pro Met Phe Lys Ser Asn Asn Arg
   420 425 430
   Tyr Arg Asp Val Ile Ala Ser Pro Asp Gly Asn Val Leu Tyr Val Leu
   435 440 445
   Thr Asp Thr Ser Gly Asn Val Gln Lys Asp Asp Gly Ser Val Thr Asn
   450 455 460
   Thr Leu Glu Asn Pro Gly Ser Leu Ile Arg Phe Thr Tyr Lys Ala Gln
   465 470 475 480
<210> 11
   <211> 1443
   <212> DNA
   <213> Acinetobacter sp.
<400> 11
   atgaataaac atttattggc taaaattact ttattaggtg ctgctcagct acttacgctc      60
   aattcagcat ttgctgatgt ccctcttaca ccatctcaat ttgctaaagc gaaaacagaa     120
   agctttgata agaaagttct tctatctaat ttaaataagc cacatgcttt gttgtgggga     180
   cctgataatc aaatttggtt aacggagcgg gcaacaggta agattctaag agttaatcca     240
   gagtcgggca gtgtaaaaac agtttttcag gttcctgaga ttgtaaatga tgctgatgga     300
   caaaacggtt tattgggttt tgcctttcat cctgacttta aaaataatcc ttatatctat     360
   gtttcaggta catttaaaaa tccgaaatct acagataaag aattaccgaa tcaaactatt     420
   atccgtcgat atacctataa caaggcaaca gatacccttg agaaaccagt agatttattg      480
   gcaggattac cttcatcgaa agaccatcag tcgggtcgtc ttgtgattgg tccagaccaa     540
   aagatttact atacgattgg tgatcaggga cgtaaccagc tggcttattt attcttacca     600
   aatcaagcac agcatacgcc gactcaacag gaactgagcg gcaaagacta tcatacctat     660
   atgggtaaag tattgcgctt aaatctggat ggaagtattc caaaagataa tccaagcttt     720
   aacggtgtaa ttagccatat ttatacgctc ggtcatcgta acccacaggg cttggcattt     780
   actccaaatg gtaaactgtt gcaatctgaa cagggtccaa actctgatga tgaaattaac     840
   ctcattgtca aaggtggtaa ctatggctgg ccaaatgtag cgggttataa agatgatagt     900
   ggttatgcct atgcaaatta ttcggcagca agcaataaag cacaaattaa agatttagga     960
   caaaatggtt taaaagtggc ggcaggtgta cctgtgatga aagagtctga atggactggt    1020
   aaaaactttg taccgccgtt aaaaacttta tataccgtcc aagataccta taactataat    1080
   gacccaactt gtggggatat gacctacatt tgctggccaa cggttgcgcc gtcatctgct    1140
   tatgtctata agggaggcaa aaaagcaatt tctggttggg aaaatacatt attggttcca    1200
   tctttaaagc gcggtgttat tttccgtatt aagctagatc caacttacag tactacttat    1260
   gatgatgctg tgccgatgtt taagagcaac aatcgttatc gtgacgtgat tgcaagtcca    1320
   gatgggaatg ttttatatgt attgactgat acttccggaa atgtccaaaa agatgatggt    1380
   tctgtaacga atacattaga aaacccagga tctctgatta gatttacata taaagctcag    1440
   taa                                                                  1443
<210> 12
   <211> 480
   <212> PRT
   <213> Acinetobacter sp.
<400> 12
   Met Asn Lys His Leu Leu Ala Lys Ile Thr Leu Leu Gly Ala Ala Gln
   1               5                   10                  15
   Leu Leu Thr Leu Asn Ser Ala Phe Ala Asp Val Pro Leu Thr Pro Ser
               20                  25                  30
   Gln Phe Ala Lys Ala Lys Thr Glu Ser Phe Asp Lys Lys Val Leu Leu
           35                  40                  45
   Ser Asn Leu Asn Lys Pro His Ala Leu Leu Trp Gly Pro Asp Asn Gln
       50                  55                  60
   Ile Trp Leu Thr Glu Arg Ala Thr Gly Lys Ile Leu Arg Val Asn Pro
   65                  70                  75                  80
   Glu Ser Gly Ser Val Lys Thr Val Phe Gln Val Pro Glu Ile Val Asn
                   85                  90                  95
   Asp Ala Asp Gly Gln Asn Gly Leu Leu Gly Phe Ala Phe His Pro Asp
               100                 105                 110
   Phe Lys Asn Asn Pro Tyr Ile Tyr Val Ser Gly Thr Phe Lys Asn Pro
           115                 120                 125
   Lys Ser Thr Asp Lys Glu Leu Pro Asn Gln Thr Ile Ile Arg Arg Tyr
       130                 135                 140
   Thr Tyr Asn Lys Ala Thr Asp Thr Leu Glu Lys Pro Val Asp Leu Leu
   145                 150                 155                 160
   Ala Gly Leu Pro Ser Ser Lys Asp His Gln Ser Gly Arg Leu Val Ile
                   165                 170                 175
   Gly Pro Asp Gln Lys Ile Tyr Tyr Thr Ile Gly Asp Gln Gly Arg Asn
               180                 185                 190
   Gln Leu Ala Tyr Leu Phe Leu Pro Asn Gln Ala Gln His Thr Pro Thr
           195                 200                 205
   Gln Gln Glu Leu Ser Gly Lys Asp Tyr His Thr Tyr Met Gly Lys Val
   210 215 220
   Leu Arg Leu Asn Leu Asp Gly Ser Ile Pro Lys Asp Asn Pro Ser Phe
   225 230 235 240
   Asn Gly Val Ile Ser His Ile Tyr Thr Leu Gly His Arg Asn Pro Gln
   245 250 255
   Gly Leu Ala Phe Thr Pro Asn Gly Lys Leu Leu Gln Ser Glu Gln Gly
   260 265 270
   Pro Asn Ser Asp Asp Glu Ile Asn Leu Ile Val Lys Gly Gly Asn Tyr
   275 280 285
   Gly Trp Pro Asn Val Ala Gly Tyr Lys Asp Asp Ser Gly Tyr Ala Tyr
   290 295 300
   Ala Asn Tyr Ser Ala Ala Ser Asn Lys Ala Gln Ile Lys Asp Leu Gly
   305 310 315 320
   Gln Asn Gly Leu Lys Val Ala Ala Gly Val Pro Val Met Lys Glu Ser
   325 330 335
   Glu Trp Thr Gly Lys Asn Phe Val Pro Pro Leu Lys Thr Leu Tyr Thr
   340 345 350
   Val Gln Asp Thr Tyr Asn Tyr Asn Asp Pro Thr Cys Gly Asp Met Thr
   355 360 365
   Tyr Ile Cys Trp Pro Thr Val Ala Pro Ser Ser Ala Tyr Val Tyr Lys
   370 375 380
   Gly Gly Lys Lys Ala Ile Ser Gly Trp Glu Asn Thr Leu Leu Val Pro
   385 390 395 400
   Ser Leu Lys Arg Gly Val Ile Phe Arg Ile Lys Leu Asp Pro Thr Tyr
   405 410 415
   Ser Thr Thr Tyr Asp Asp Ala Val Pro Met Phe Lys Ser Asn Asn Arg
   420 425 430
   Tyr Arg Asp Val Ile Ala Ser Pro Asp Gly Asn Val Leu Tyr Val Leu
   435 440 445
   Thr Asp Thr Ser Gly Asn Val Gln Lys Asp Asp Gly Ser Val Thr Asn
   450 455 460
   Thr Leu Glu Asn Pro Gly Ser Leu Ile Arg Phe Thr Tyr Lys Ala Gln
   465 470 475 480
<210> 13
   <211> 1443
   <212> DNA
   <213> Acinetobacter sp.
<400> 13
   atgaataaac atttattggc taaaattact ttattaggtg ctgctcagct agttacgctc 60
   aattcagcat ttgctgatgt ccctcttact ccatctcaat ttgctaaagc gaaaacagga 120
   agctttgaca agaaagttct tatatctaat ttaaataagc cacatgcttt gttgtggggg 180
   cctgataatc aaatttggtt aacggagcgg gcaacaggta agattctaag agttaatcca 240
   gagtcgggca gtgtaaaaac agtttttcag gttcctgaga ttgtaaatga tgctgatgga 300
   caaaacggtt tattgggttt tgcctttcat cctgacttta aaaataatcc ttatatctat 360
   gtttcaggta catttaaaaa tccgaaatct acagataaag aattaccgaa tcaaactatt 420
   atccgtcgat atacctataa caaagcaaca gatactcttg agaaaccagt agatttattg 480
   gcaggattac cttcatcgaa agaccatcag tcgggtcgcc ttgtcattgg tccagaccaa 540
   aagatttact atacgattgg tgatcaggga cgtaaccagc ttgcttattt attcttacca 600
   aatcaggcac aacatacgcc gactcaacag gaactgagcg gaaaagacta tcatacctat 660
   atgggtaaag tattacgctt aaatctggat ggaagtattc caaaagataa tccaagcttt 720
   aacggtgtaa ttagtcatat ttatacgctc ggtcatcgta atccacaggg cttggcattt 780
   actccaaatg gtaaactgtt gcaatctgaa caaggtccaa actctgatga tgaaattaat 840
   ctcattgtta aaggtggcaa ctatggctgg ccaaatgtag cgggttataa agatgacagt 900
   ggttatgcct atgcaaatta ttcggcagca gccaataagt cacaaattaa agatttaggg 960
   caaaatggag taaaagtagc ggcaggtgta cctgtgatga aagagtctga atggactggt 1020
   aaaaactttg taccgccgtt aaaaacttta tataccgtcc aagataccta taactataat 1080
   gacccaacct gtggggatat gacctacatt tgctggccaa cagttgcacc gtcatctgct 1140
   tatgtctata agggcggcaa aaaagcaatt tctggttggg aaaatacatt attggttcca 1200
   tctttaaagc gtggtgttat tttccgtatt aagctagatc caacttacag tgctacttat 1260
   gatgatgctg tgccgatgtt taagagcaac aatcgttatc gtgacgtgat tgcaagtcca 1320
   gatgggaatg ttttatatgt attgactgat acttccggaa atgtccaaaa agatgatggt 1380
   tctgtaacga atacattaga aaatccagga tctctgatta gatttacata taaagctcag 1440
   taa 1443
<210> 14
   <211> 480
   <212> PRT
   <213> Acinetobacter sp.
<400> 14
   Met Asn Lys His Leu Leu Ala Lys Ile Thr Leu Leu Gly Ala Ala Gln
   1 5 10 15
   Leu Val Thr Leu Asn Ser Ala Phe Ala Asp Val Pro Leu Thr Pro Ser
   20 25 30
   Gln Phe Ala Lys Ala Lys Thr Gly Ser Phe Asp Lys Lys Val Leu Ile
   35 40 45
   Ser Asn Leu Asn Lys Pro His Ala Leu Leu Trp Gly Pro Asp Asn Gln
   50 55 60
   Ile Trp Leu Thr Glu Arg Ala Thr Gly Lys Ile Leu Arg Val Asn Pro
   65 70 75 80
   Glu Ser Gly Ser Val Lys Thr Val Phe Gln Val Pro Glu Ile Val Asn
   85 90 95
   Asp Ala Asp Gly Gln Asn Gly Leu Leu Gly Phe Ala Phe His Pro Asp
   100 105 110
   Phe Lys Asn Asn Pro Tyr Ile Tyr Val Ser Gly Thr Phe Lys Asn Pro
   115 120 125
   Lys Ser Thr Asp Lys Glu Leu Pro Asn Gln Thr Ile Ile Arg Arg Tyr
   130 135 140
   Thr Tyr Asn Lys Ala Thr Asp Thr Leu Glu Lys Pro Val Asp Leu Leu
   145 150 155 160
   Ala Gly Leu Pro Ser Ser Lys Asp His Gln Ser Gly Arg Leu Val Ile
   165 170 175
   Gly Pro Asp Gln Lys Ile Tyr Tyr Thr Ile Gly Asp Gln Gly Arg Asn
   180 185 190
   Gln Leu Ala Tyr Leu Phe Leu Pro Asn Gln Ala Gln His Thr Pro Thr
   195 200 205
   Gln Gln Glu Leu Ser Gly Lys Asp Tyr His Thr Tyr Met Gly Lys Val
   210 215 220
   Leu Arg Leu Asn Leu Asp Gly Ser Ile Pro Lys Asp Asn Pro Ser Phe
   225 230 235 240
   Asn Gly Val Ile Ser His Ile Tyr Thr Leu Gly His Arg Asn Pro Gln
   245 250 255
   Gly Leu Ala Phe Thr Pro Asn Gly Lys Leu Leu Gln Ser Glu Gln Gly
   260 265 270
   Pro Asn Ser Asp Asp Glu Ile Asn Leu Ile Val Lys Gly Gly Asn Tyr
   275 280 285
   Gly Trp Pro Asn Val Ala Gly Tyr Lys Asp Asp Ser Gly Tyr Ala Tyr
   290 295 300
   Ala Asn Tyr Ser Ala Ala Ala Asn Lys Ser Gln Ile Lys Asp Leu Gly
   305 310 315 320
   Gln Asn Gly Val Lys Val Ala Ala Gly Val Pro Val Met Lys Glu Ser
   325 330 335
   Glu Trp Thr Gly Lys Asn Phe Val Pro Pro Leu Lys Thr Leu Tyr Thr
   340 345 350
   Val Gln Asp Thr Tyr Asn Tyr Asn Asp Pro Thr Cys Gly Asp Met Thr
   355 360 365
   Tyr Ile Cys Trp Pro Thr Val Ala Pro Ser Ser Ala Tyr Val Tyr Lys
   370 375 380
   Gly Gly Lys Lys Ala Ile Ser Gly Trp Glu Asn Thr Leu Leu Val Pro
   385 390 395 400
   Ser Leu Lys Arg Gly Val Ile Phe Arg Ile Lys Leu Asp Pro Thr Tyr
   405 410 415
   Ser Ala Thr Tyr Asp Asp Ala Val Pro Met Phe Lys Ser Asn Asn Arg
   420 425 430
   Tyr Arg Asp Val Ile Ala Ser Pro Asp Gly Asn Val Leu Tyr Val Leu
   435 440 445
   Thr Asp Thr Ser Gly Asn Val Gln Lys Asp Asp Gly Ser Val Thr Asn
   450 455 460
   Thr Leu Glu Asn Pro Gly Ser Leu Ile Arg Phe Thr Tyr Lys Ala Gln
   465 470 475 480
<210> 15
   <211> 1443
   <212> DNA
   <213> Acinetobacter sp.
<400> 15
   atgaataaac atttattggc taaaattact ttattaggtg ctgctcagtt acttacgctc      60
   aattcagcat ttgctgatgt ccctcttacc ccatctcaat ttgctaaagc gaaaacagaa     120
   agctttgaca agaaagttct tctatctaat ttaaataagc cacatgcttt gttgtgggga     180
   cctgataatc aaatttggtt aacggagcgg gcaacaggta agattctaag agttaatcca     240
   gagtcgggca gtgtaaaaac agtttttcag gttcctgaga ttgtaagtga tgctgatgga     300
   caaaacggtt tattgggttt tgcctttcat actgacttta aaaataatcc ttatatctat     360
   gtttcaggta catttaaaaa tccgaaatct acagataaag aattaccgaa tcaaactatt     420
   atccgtcgat atacctataa caaagcaaca gatactcttg agaaaccagt agatttatta     480
   gcaggattac cttcatcgaa agaccatcag tcgggtcgtc ttgtcattgg tccagaccaa     540
   aagatttact atacgattgg tgatcaggga cgtaaccagc ttgcttattt attcttacca     600
   aatcaggcac aacatacgcc gactcaacag gaactgagcg gcaaagacta tcatacctat     660
   atgggtaaag tattacgctt aaatctggat ggaagtattc caaaagataa tccaagcttt     720
   aacggtgtaa ttagccatat ttatacgctc ggtcatcgta acccacaggg cttggcattt     780
   actccaaatg gtaaactgtt gcaatctgaa cagggtccaa actctgacga tgaaattaat     840
   ctcattgtta aaggtggtaa ctatggctgg ccaaatgtag cgggttataa agatgacagt     900
   ggttatgcct atgcaaatta ttcggcagca accaataagt cacaaattaa agatttaggg     960
   caaaatggag taaaagtagc ggcaggtgta cctgtgatga aagagtctga atggactggt    1020
   aaaaactttg taccgccgtt aaaaacttta tataccgtcc aagataccta taactataat    1080
   gacccaactt gtggggatat gacctacatt tgctggccaa cggttgcgcc gtcatctgct   1140
   tatgtctata agggaggcaa aaaagcaatt tctggttggg aaaatacatt attggttcca   1200
   tctttaaagc gcggtgttat tttccgtatt aagctagatc caacttacag tgctacttat   1260
   gatgatgctg tgccgatgtt taaaagcaac aatcgttatc gtgacgtgat tgcaagtcca   1320
   gatgggaatg ttttatatgt attgactgat acttccggaa atgtccaaaa agatgatggt   1380
   tctgtaacga atacattaga aaatccagga tccctgatta gatttacata taaagctcag   1440
   taa                                                                 1443
<210> 16
   <211> 480
   <212> PRT
   <213> Acinetobacter sp.
<400> 16
   Met Asn Lys His Leu Leu Ala Lys Ile Thr Leu Leu Gly Ala Ala Gln
   1               5                   10                  15
   Leu Leu Thr Leu Asn Ser Ala Phe Ala Asp Val Pro Leu Thr Pro Ser
               20                  25                  30
   Gln Phe Ala Lys Ala Lys Thr Glu Ser Phe Asp Lys Lys Val Leu Leu
           35                  40                  45
   Ser Asn Leu Asn Lys Pro His Ala Leu Leu Trp Gly Pro Asp Asn Gln
       50                  55                  60
   Ile Trp Leu Thr Glu Arg Ala Thr Gly Lys Ile Leu Arg Val Asn Pro
   65                  70                  75                  80
   Glu Ser Gly Ser Val Lys Thr Val Phe Gln Val Pro Glu Ile Val Ser
                   85                  90                  95
   Asp Ala Asp Gly Gln Asn Gly Leu Leu Gly Phe Ala Phe His Thr Asp
               100                 105                 110
   Phe Lys Asn Asn Pro Tyr Ile Tyr Val Ser Gly Thr Phe Lys Asn Pro
           115                 120                 125
   Lys Ser Thr Asp Lys Glu Leu Pro Asn Gln Thr Ile Ile Arg Arg Tyr
       130                 135                 140
   Thr Tyr Asn Lys Ala Thr Asp Thr Leu Glu Lys Pro Val Asp Leu Leu
   145 150 155 160
   Ala Gly Leu Pro Ser Ser Lys Asp His Gln Ser Gly Arg Leu Val Ile
   165 170 175
   Gly Pro Asp Gln Lys Ile Tyr Tyr Thr Ile Gly Asp Gln Gly Arg Asn
   180 185 190
   Gln Leu Ala Tyr Leu Phe Leu Pro Asn Gln Ala Gln His Thr Pro Thr
   195 200 205
   Gln Gln Glu Leu Ser Gly Lys Asp Tyr His Thr Tyr Met Gly Lys Val
   210 215 220
   Leu Arg Leu Asn Leu Asp Gly Ser Ile Pro Lys Asp Asn Pro Ser Phe
   225 230 235 240
   Asn Gly Val Ile Ser His Ile Tyr Thr Leu Gly His Arg Asn Pro Gln
   245 250 255
   Gly Leu Ala Phe Thr Pro Asn Gly Lys Leu Leu Gln Ser Glu Gln Gly
   260 265 270
   Pro Asn Ser Asp Asp Glu Ile Asn Leu Ile Val Lys Gly Gly Asn Tyr
   275 280 285
   Gly Trp Pro Asn Val Ala Gly Tyr Lys Asp Asp Ser Gly Tyr Ala Tyr
   290 295 300
   Ala Asn Tyr Ser Ala Ala Thr Asn Lys Ser Gln Ile Lys Asp Leu Gly
   305 310 315 320
   Gln Asn Gly Val Lys Val Ala Ala Gly Val Pro Val Met Lys Glu Ser
   325 330 335
   Glu Trp Thr Gly Lys Asn Phe Val Pro Pro Leu Lys Thr Leu Tyr Thr
   340 345 350
   Val Gln Asp Thr Tyr Asn Tyr Asn Asp Pro Thr Cys Gly Asp Met Thr
   355 360 365
   Tyr Ile Cys Trp Pro Thr Val Ala Pro Ser Ser Ala Tyr Val Tyr Lys
   370 375 380
   Gly Gly Lys Lys Ala Ile Ser Gly Trp Glu Asn Thr Leu Leu Val Pro
   385 390 395 400
   Ser Leu Lys Arg Gly Val Ile Phe Arg Ile Lys Leu Asp Pro Thr Tyr
   405 410 415
   Ser Ala Thr Tyr Asp Asp Ala Val Pro Met Phe Lys Ser Asn Asn Arg
   420 425 430
   Tyr Arg Asp Val Ile Ala Ser Pro Asp Gly Asn Val Leu Tyr Val Leu
   435 440 445
   Thr Asp Thr Ser Gly Asn Val Gln Lys Asp Asp Gly Ser Val Thr Asn
   450 455 460
   Thr Leu Glu Asn Pro Gly Ser Leu Ile Arg Phe Thr Tyr Lys Ala Gln
   465 470 475 480
<210> 17
   <211> 1443
   <212> DNA
   <213> Acinetobacter sp.
<400> 17
   atgaataaac atttattggc taaaattact ttattaggtg ctgctcagct acttacgctc 60
   aattcagcat ttgctgatgt ccctcttact ccatctcaat ttgctaaagc gaaaacagaa 120
   agctttgaca agaaagttct tctatctaat ttaaataagc cacatgcttt gttgtgggga 180
   cctgataatc aaatttggtt aacggagcgg gcaacaggta agattctaag agttaaccct 240
   gaatcaggca gtgtaaaaac agtttttcag gttcctgaga ttgtaaatga tgctgatgga 300
   caaaacggtt tattgggttt tgcctttcat cctgacttta aacataatcc ttatatctat 360
   gtttcaggta catttaaaaa tccgaaatct acagataaag aattaccgaa tcaaactatt 420
   atccgtcgat atacctataa caaggcaaca gatacccttg agaaaccagt agatttatta 480
   gcaggattac cttcatcgaa agaccatcag tcgggtcgtc ttgtgattgg tccagaccaa 540
   aagatttact atacgattgg tgatcagggg cgtaaccagc tggcttattt attcttgcca 600
   aatcaagcac agcatacgcc gactcaacaa gagctgagcg gtaaagacta tcacacctat 660
   atgggtaaag tattacgctt aaatctagat ggaagtattc caaaagataa tccaagcttt 720
   aacggtgtaa ttagccatat ttatacactc ggtcatcgta atccacaggg cttggccttt 780
   actccaaatg gtaaactgtt gcaatctgaa caaggtccaa actctgatga tgaaattaat 840
   ctgattgtta aaggtggtaa ctatggctgg ccaaatgtag cgggttataa agatgacagt     900
   ggttatgcct atgcaaatta ttcggcagca accaataagt cacaaattaa agatttaggg     960
   caaaatggag taaaagtagc ggcaggtgta cctgtaatga aagagtctga atggactggt    1020
   aaaaactttg taccgccgtt aaaaacttta tataccgtcc aagataccta taactataat    1080
   gacccaacct gtggggatat gacctacatt tgctggccaa cggttgcgcc gtcatctgct    1140
   tatgtctata agggaggcaa aaaagcaatt tctggttggg aaaatacctt attggttcca    1200
   tctttaaagc gcggtgttat tttccgtatt aagctagatc caacttacag tgctacttat    1260
   gatgatgctg tgccgatgtt taagagcaac aatcgttatc gtgacgtgat tgcaagtcca    1320
   gatggaaatg ttttatatgt attgactgat acttccggaa atgtccaaaa agatgatggc    1380
   tctgtaacga atacattaga aaatccagga tctttgatta ggtttacata taaagctcag    1440
   taa                                                                  1443
<210> 18
   <211> 480
   <212> PRT
   <213> Acinetobacter sp.
<400> 18
   Met Asn Lys His Leu Leu Ala Lys Ile Thr Leu Leu Gly Ala Ala Gln
   1               5                   10                  15
   Leu Leu Thr Leu Asn Ser Ala Phe Ala Asp Val Pro Leu Thr Pro Ser
               20                  25                  30
   Gln Phe Ala Lys Ala Lys Thr Glu Ser Phe Asp Lys Lys Val Leu Leu
           35                  40                  45
   Ser Asn Leu Asn Lys Pro His Ala Leu Leu Trp Gly Pro Asp Asn Gln
       50                  55                  60
   Ile Trp Leu Thr Glu Arg Ala Thr Gly Lys Ile Leu Arg Val Asn Pro
   65                  70                  75                  80
   Glu Ser Gly Ser Val Lys Thr Val Phe Gln Val Pro Glu Ile Val Asn
                   85                  90                  95
   Asp Ala Asp Gly Gln Asn Gly Leu Leu Gly Phe Ala Phe His Pro Asp
               100                 105                 110
   Phe Lys His Asn Pro Tyr Ile Tyr Val Ser Gly Thr Phe Lys Asn Pro
           115                 120                 125
   Lys Ser Thr Asp Lys Glu Leu Pro Asn Gln Thr Ile Ile Arg Arg Tyr
       130                 135                 140
   Thr Tyr Asn Lys Ala Thr Asp Thr Leu Glu Lys Pro Val Asp Leu Leu
   145                 150                 155                 160
   Ala Gly Leu Pro Ser Ser Lys Asp His Gln Ser Gly Arg Leu Val Ile
                   165                 170                 175
   Gly Pro Asp Gln Lys Ile Tyr Tyr Thr Ile Gly Asp Gln Gly Arg Asn
               180                 185                 190
   Gln Leu Ala Tyr Leu Phe Leu Pro Asn Gln Ala Gln His Thr Pro Thr
           195                 200                 205
   Gln Gln Glu Leu Ser Gly Lys Asp Tyr His Thr Tyr Met Gly Lys Val
       210                 215                 220
   Leu Arg Leu Asn Leu Asp Gly Ser Ile Pro Lys Asp Asn Pro Ser Phe
   225                 230                 235                 240
   Asn Gly Val Ile Ser His Ile Tyr Thr Leu Gly His Arg Asn Pro Gln
                   245                 250                 255
   Gly Leu Ala Phe Thr Pro Asn Gly Lys Leu Leu Gln Ser Glu Gln Gly
               260                 265                 270
   Pro Asn Ser Asp Asp Glu Ile Asn Leu Ile Val Lys Gly Gly Asn Tyr
           275                 280                 285
   Gly Trp Pro Asn Val Ala Gly Tyr Lys Asp Asp Ser Gly Tyr Ala Tyr
       290                 295                 300
   Ala Asn Tyr Ser Ala Ala Thr Asn Lys Ser Gln Ile Lys Asp Leu Gly
   305                 310                 315                 320
   Gln Asn Gly Val Lys Val Ala Ala Gly Val Pro Val Met Lys Glu Ser
                   325                 330                 335
   Glu Trp Thr Gly Lys Asn Phe Val Pro Pro Leu Lys Thr Leu Tyr Thr
               340                 345                 350
   Val Gln Asp Thr Tyr Asn Tyr Asn Asp Pro Thr Cys Gly Asp Met Thr
   355 360 365
   Tyr Ile Cys Trp Pro Thr Val Ala Pro Ser Ser Ala Tyr Val Tyr Lys
   370 375 380
   Gly Gly Lys Lys Ala Ile Ser Gly Trp Glu Asn Thr Leu Leu Val Pro
   385 390 395 400
   Ser Leu Lys Arg Gly Val Ile Phe Arg Ile Lys Leu Asp Pro Thr Tyr
   405 410 415
   Ser Ala Thr Tyr Asp Asp Ala Val Pro Met Phe Lys Ser Asn Asn Arg
   420 425 430
   Tyr Arg Asp Val Ile Ala Ser Pro Asp Gly Asn Val Leu Tyr Val Leu
   435 440 445
   Thr Asp Thr Ser Gly Asn Val Gln Lys Asp Asp Gly Ser Val Thr Asn
   450 455 460
   Thr Leu Glu Asn Pro Gly Ser Leu Ile Arg Phe Thr Tyr Lys Ala Gln
   465 470 475 480
<210> 19
   <211> 1443
   <212> DNA
   <213> Acinetobacter sp.
<400> 19
   atgaataaac atttattggc taaaattact ttattaggtg ctgctcacct acttacgctc 60
   aattcagcat ttgctgatgt ccctcttact ccatctcaat ttgctaaagc gaaaacagaa 120
   agctttgaca agaaagttct tctatctaat ttaaataagc cgcatgcttt gttgtgggga 180
   cctgataatc aaatttggtt aacagagcgg gcaacaggta agattctaag agttaaccct 240
   gaatcaggca gtgtaaaaac agtttttcag gttcctgaga ttgtaaatga tgctgatgga 300
   caaaacggtt tattgggttt tgcctttcat cctgacttta aacataatcc ttatatctat 360
   gtttcaggta catttaaaaa tccgaaatct acagataaag aattaccgaa tcaaactatt 420
   attcgtcgat atacctataa caaagcaaca gatactcttg ataaaccagt agatttatta 480
   gcaggattac cttcatcgaa agaccatcag tcgggtcgtc ttgtgattgg tccagaccaa 540
   aagatttact atacgattgg tgatcagggg cgtaaccagc tggcttattt attcttacca 600
   aatcaggcac aacatacgcc gactcaacag gaactgagcg gcaaagacta tcatacctat     660
   atgggtaaag tattacgctt aaatctggat ggaagtattc caaaagataa tccaagcttt     720
   aacggtgtaa ttagccatat ttatacgctc ggtcatcgta acccacaggg cttggcattt     780
   actccaaatg gtaaactgtt acaatctgaa cagggtccaa actctgacga tgaaattaat     840
   ctcattgtta aaggtggtaa ctatggctgg ccaaatgtag cgggttataa agatgacagt     900
   ggttatgcgt atgcaaatta ttcagcagca accaataagt cacaaattaa agatttaggg     960
   caaaatggag taaaagtagc ggcaggtgta cctgtgatga aagagtctga atggactggt    1020
   aaaaactttg taccgccgtt aaaaacttta tataccgtcc aagataccta taactataat    1080
   gacccaactt gtggggatat gacctacatt tgctggccaa cagttgcgcc atcatctgct    1140
   tatgtctata aggggggcaa aaaagcaatt tctggttggg aaaatacatt attggttcca    1200
   tctttaaagc gtggtgttat ttttcgtatt aagctagatc caacttacag tactacttat    1260
   gatgatgctg tgccgatgtt taagagcaac aatcgttatc gtgacgtgat tgcaagtcca    1320
   gatgggaatg ttttatatgt attgactgat acttccggaa atgtccaaaa agatgatggt    1380
   tctgtaacga atacattaga aaacccagga tctctgatta gatttacata taaagctcag    1440
   taa                                                                  1443
<210> 20
   <211> 480
   <212> PRT
   <213> Acinetobacter sp.
<400> 20
   Met Asn Lys His Leu Leu Ala Lys Ile Thr Leu Leu Gly Ala Ala His
   1               5                   10                  15
   Leu Leu Thr Leu Asn Ser Ala Phe Ala Asp Val Pro Leu Thr Pro Ser
               20                  25                  30
   Gln Phe Ala Lys Ala Lys Thr Glu Ser Phe Asp Lys Lys Val Leu Leu
           35                  40                  45
   Ser Asn Leu Asn Lys Pro His Ala Leu Leu Trp Gly Pro Asp Asn Gln
       50                  55                  60
   Ile Trp Leu Thr Glu Arg Ala Thr Gly Lys Ile Leu Arg Val Asn Pro
   65                  70                  75                  80
   Glu Ser Gly Ser Val Lys Thr Val Phe Gln Val Pro Glu Ile Val Asn
   85 90 95
   Asp Ala Asp Gly Gln Asn Gly Leu Leu Gly Phe Ala Phe His Pro Asp
   100 105 110
   Phe Lys His Asn Pro Tyr Ile Tyr Val Ser Gly Thr Phe Lys Asn Pro
   115 120 125
   Lys Ser Thr Asp Lys Glu Leu Pro Asn Gln Thr Ile Ile Arg Arg Tyr
   130 135 140
   Thr Tyr Asn Lys Ala Thr Asp Thr Leu Asp Lys Pro Val Asp Leu Leu
   145 150 155 160
   Ala Gly Leu Pro Ser Ser Lys Asp His Gln Ser Gly Arg Leu Val Ile
   165 170 175
   Gly Pro Asp Gln Lys Ile Tyr Tyr Thr Ile Gly Asp Gln Gly Arg Asn
   180 185 190
   Gln Leu Ala Tyr Leu Phe Leu Pro Asn Gln Ala Gln His Thr Pro Thr
   195 200 205
   Gln Gln Glu Leu Ser Gly Lys Asp Tyr His Thr Tyr Met Gly Lys Val
   210 215 220
   Leu Arg Leu Asn Leu Asp Gly Ser Ile Pro Lys Asp Asn Pro Ser Phe
   225 230 235 240
   Asn Gly Val Ile Ser His Ile Tyr Thr Leu Gly His Arg Asn Pro Gln
   245 250 255
   Gly Leu Ala Phe Thr Pro Asn Gly Lys Leu Leu Gln Ser Glu Gln Gly
   260 265 270
   Pro Asn Ser Asp Asp Glu Ile Asn Leu Ile Val Lys Gly Gly Asn Tyr
   275 280 285
   Gly Trp Pro Asn Val Ala Gly Tyr Lys Asp Asp Ser Gly Tyr Ala Tyr
   290 295 300
   Ala Asn Tyr Ser Ala Ala Thr Asn Lys Ser Gln Ile Lys Asp Leu Gly
   305 310 315 320
   Gln Asn Gly Val Lys Val Ala Ala Gly Val Pro Val Met Lys Glu Ser
   325 330 335
   Glu Trp Thr Gly Lys Asn Phe Val Pro Pro Leu Lys Thr Leu Tyr Thr
   340 345 350
   Val Gln Asp Thr Tyr Asn Tyr Asn Asp Pro Thr Cys Gly Asp Met Thr
   355 360 365
   Tyr Ile Cys Trp Pro Thr Val Ala Pro Ser Ser Ala Tyr Val Tyr Lys
   370 375 380
   Gly Gly Lys Lys Ala Ile Ser Gly Trp Glu Asn Thr Leu Leu Val Pro
   385 390 395 400
   Ser Leu Lys Arg Gly Val Ile Phe Arg Ile Lys Leu Asp Pro Thr Tyr
   405 410 415
   Ser Thr Thr Tyr Asp Asp Ala Val Pro Met Phe Lys Ser Asn Asn Arg
   420 425 430
   Tyr Arg Asp Val Ile Ala Ser Pro Asp Gly Asn Val Leu Tyr Val Leu
   435 440 445
   Thr Asp Thr Ser Gly Asn Val Gln Lys Asp Asp Gly Ser Val Thr Asn
   450 455 460
   Thr Leu Glu Asn Pro Gly Ser Leu Ile Arg Phe Thr Tyr Lys Ala Gln
   465 470 475 480
<210> 21
   <211> 1443
   <212> DNA
   <213> Acinetobacter sp.
<400> 21
   atgaataaac atttattggc taaaattact ttattaggtg ctgctcagct acttacgctc 60
   aattcagcat ttgctgatgt ccctcttaca ccatctcaat ttgctaaagc gaaaacagaa 120
   agctttgaca agaaagttct tctatctaat ttaaataagc cacatgcttt gttgtggggg 180
   cctgataatc aaatttggtt aacggagcgg gcaacaggta agattctaag agtgaatcca 240
   gagtcgggca gtgtaaaaac agtttttcag gttcctgaga ttgtaaatga tgctgatgga 300
   caaaacggtt tattgggttt tgcctttcat cctgacttta aaaataatcc ttatatctat 360
   gtttcaggta cttttaaaaa tccgaaatct acagataaag aattaccgaa tcaaactatt 420
   attcgtcgat atacctataa caaagcaaca gatactcttg ataaaccagt agatttatta 480
   gcaggattac cttcatcgaa agaccatcag tcgggtcgcc ttgtgattgg tccagaccaa 540
   aagatttact atacgattgg tgatcagggg cgtaaccagc ttgcttattt attcttacca 600
   aatcaggcac aacatacgcc gactcaacag gaactgagcg gcaaagacta tcatacctat 660
   atgggtaaag tattacgctt aaatctggat ggaagtattc caaaagataa tccaagcttt 720
   aacggtgtaa ttagccatat ttatacgctc ggtcatcgta acccacaggg cttggcattt 780
   actccaaatg gtaaactgtt gcaatctgaa cagggtccaa actctgatga tgaaattaac 840
   ctcattgtca aaggtggtaa ctatggctgg ccaaatgtag cgggttataa agatgatagt 900
   ggttatgcct atgcaaatta ttcagcagca agcaataaag cacaaattaa agatttagga 960
   caaaatggtt taaaagtggc agctggcgtt ccagtgacta aagagtctga atggactggt 1020
   aaaaactttg taccgccgtt aaaaacttta tataccgtcc aagataccta taactataat 1080
   gacccaacct gtggggatat gacctacatt tgctggccaa cggttgcgcc gtcatctgct 1140
   tatgtctata agggaggcaa aaaagcaatt tctggttggg aaaatacctt attggttcca 1200
   tctttaaagc gcggtgttat tttccgtatt aagctagatc caacttacag tgctacttat 1260
   gatgatgctg tgccgatgtt taagagcaac aatcgttatc gtgacgtgat tgcaagtcca 1320
   gatggaaatg ttttatatgt attgactgat acttccggaa atgtccaaaa agatgatggt 1380
   tctgtaacga atacattaga aaacccagga tctctgatta gatttacata taaagctcag 1440
   taa 1443
<210> 22
   <211> 480
   <212> PRT
   <213> Acinetobacter sp.
<400> 22
   Met Asn Lys His Leu Leu Ala Lys Ile Thr Leu Leu Gly Ala Ala Gln
   1               5                   10                  15
   Leu Leu Thr Leu Asn Ser Ala Phe Ala Asp Val Pro Leu Thr Pro Ser
               20                  25                  30
   Gln Phe Ala Lys Ala Lys Thr Glu Ser Phe Asp Lys Lys Val Leu Leu
           35                  40                  45
   Ser Asn Leu Asn Lys Pro His Ala Leu Leu Trp Gly Pro Asp Asn Gln
   50 55 60
   Ile Trp Leu Thr Glu Arg Ala Thr Gly Lys Ile Leu Arg Val Asn Pro
   65 70 75 80
   Glu Ser Gly Ser Val Lys Thr Val Phe Gln Val Pro Glu Ile Val Asn
   85 90 95
   Asp Ala Asp Gly Gln Asn Gly Leu Leu Gly Phe Ala Phe His Pro Asp
   100 105 110
   Phe Lys Asn Asn Pro Tyr Ile Tyr Val Ser Gly Thr Phe Lys Asn Pro
   115 120 125
   Lys Ser Thr Asp Lys Glu Leu Pro Asn Gln Thr Ile Ile Arg Arg Tyr
   130 135 140
   Thr Tyr Asn Lys Ala Thr Asp Thr Leu Asp Lys Pro Val Asp Leu Leu
   145 150 155 160
   Ala Gly Leu Pro Ser Ser Lys Asp His Gln Ser Gly Arg Leu Val Ile
   165 170 175
   Gly Pro Asp Gln Lys Ile Tyr Tyr Thr Ile Gly Asp Gln Gly Arg Asn
   180 185 190
   Gln Leu Ala Tyr Leu Phe Leu Pro Asn Gln Ala Gln His Thr Pro Thr
   195 200 205
   Gln Gln Glu Leu Ser Gly Lys Asp Tyr His Thr Tyr Met Gly Lys Val
   210 215 220
   Leu Arg Leu Asn Leu Asp Gly Ser Ile Pro Lys Asp Asn Pro Ser Phe
   225 230 235 240
   Asn Gly Val Ile Ser His Ile Tyr Thr Leu Gly His Arg Asn Pro Gln
   245 250 255
   Gly Leu Ala Phe Thr Pro Asn Gly Lys Leu Leu Gln Ser Glu Gln Gly
   260 265 270
   Pro Asn Ser Asp Asp Glu Ile Asn Leu Ile Val Lys Gly Gly Asn Tyr
   275 280 285
   Gly Trp Pro Asn Val Ala Gly Tyr Lys Asp Asp Ser Gly Tyr Ala Tyr
   290 295 300
   Ala Asn Tyr Ser Ala Ala Ser Asn Lys Ala Gln Ile Lys Asp Leu Gly
   305 310 315 320
   Gln Asn Gly Leu Lys Val Ala Ala Gly Val Pro Val Thr Lys Glu Ser
   325 330 335
   Glu Trp Thr Gly Lys Asn Phe Val Pro Pro Leu Lys Thr Leu Tyr Thr
   340 345 350
   Val Gln Asp Thr Tyr Asn Tyr Asn Asp Pro Thr Cys Gly Asp Met Thr
   355 360 365
   Tyr Ile Cys Trp Pro Thr Val Ala Pro Ser Ser Ala Tyr Val Tyr Lys
   370 375 380
   Gly Gly Lys Lys Ala Ile Ser Gly Trp Glu Asn Thr Leu Leu Val Pro
   385 390 395 400
   Ser Leu Lys Arg Gly Val Ile Phe Arg Ile Lys Leu Asp Pro Thr Tyr
   405 410 415
   Ser Ala Thr Tyr Asp Asp Ala Val Pro Met Phe Lys Ser Asn Asn Arg
   420 425 430
   Tyr Arg Asp Val Ile Ala Ser Pro Asp Gly Asn Val Leu Tyr Val Leu
   435 440 445
   Thr Asp Thr Ser Gly Asn Val Gln Lys Asp Asp Gly Ser Val Thr Asn
   450 455 460
   Thr Leu Glu Asn Pro Gly Ser Leu Ile Arg Phe Thr Tyr Lys Ala Gln
   465 470 475 480
<210> 23
   <211> 1443
   <212> DNA
   <213> Acinetobacter sp.
<400> 23
   atgaataaac atttattggc taaaattact ttattaggtg ctgctcagct acttacactc 60
   aattcagcat ttgctgatgt ccctcttaca ccatctcaat ttgctaaagc gaaaacagaa 120
   agctttgaca agaaagttct tctatctaat ttaaataagc cacatgcttt gttgtggggg    180
   cctgataatc aaatttggtt aacggagcgg gcaacaggta agattctaag agtgaatcca    240
   gagtcgggca gtgtaaaaac agtttttcag gttcctgaga ttgtaaatga tgctgatgga    300
   caaaacggtt tattgggttt tgcctttcat cctgacttta aaaataatcc ttatatctat    360
   gtttcaggta cttttaaaaa tccgaaatct acagataaag aattaccgaa tcaaactatt    420
   attcgtcgat atacctataa caaagcaaca gatactcttg ataaaccagt agatttatta    480
   gcaggattac cttcatcgaa agaccatcag tcgggtcgcc ttgtgattgg tccagaccaa    540
   aaaatttact atacgattgg tgatcagggg cgtaaccagc ttgcttattt attcttacca    600
   aatcaggcac aacatacgcc gactcaacag gaactgagcg gcaaagacta tcatacctat    660
   atgggtaaag tattacgctt aaatctggat ggaagtattc caaaagataa tccaagcttt    720
   aacggtgtaa ttagtcatat ttatacgctc ggtcatcgta atccacaggg cttagcattc    780
   actccaaatg gtaaactgtt gcaatctgaa caaggtccaa actctgatga tgaaattaat    840
   ctcattgtta aaggtggtaa ctatggctgg ccaaatgtag ctggttataa agatgatagt    900
   ggttatgcct atgcaaatta ttcagcagca agcaataaag cacaaattaa agatttagga    960
   caaaatggtt taaaagtggc agctggcgtt ccagtgacta aagagtctga atggactggt   1020
   aaaaactttg taccgccgtt aaaaacttta tataccgtcc aagataccta taactataat   1080
   gacccaacct gtggggatat gacctacatt tgctggccaa cggttgcgcc gtcatctgct   1140
   tatgtctata agggaggcaa aaaagcaatt tctggttggg aaaatacctt attggttcca   1200
   tctttaaagc gcggtgttat tttccgtatt aagctagatc caacttacaa tactacttat   1260
   gatgatgctg tgccgatgtt taagagcaac aatcgttatc gtgacgtgat tgcaagtcca   1320
   gatggaaatg ttttatatgt attgactgat acttccggaa atgtccaaaa agatgatggc   1380
   tctgtaacga atacattaga aaacccagga tctctgatta gatttacata taaagctcag   1440
   taa                                                                 1443
<210> 24
   <211> 480
   <212> PRT
   <213> Acinetobacter sp.
<400> 24
   Met Asn Lys His Leu Leu Ala Lys Ile Thr Leu Leu Gly Ala Ala Gln
   1               5                   10                  15
   Leu Leu Thr Leu Asn Ser Ala Phe Ala Asp Val Pro Leu Thr Pro Ser
   20 25 30
   Gln Phe Ala Lys Ala Lys Thr Glu Ser Phe Asp Lys Lys Val Leu Leu
   35 40 45
   Ser Asn Leu Asn Lys Pro His Ala Leu Leu Trp Gly Pro Asp Asn Gln
   50 55 60
   Ile Trp Leu Thr Glu Arg Ala Thr Gly Lys Ile Leu Arg Val Asn Pro
   65 70 75 80
   Glu Ser Gly Ser Val Lys Thr Val Phe Gln Val Pro Glu Ile Val Asn
   85 90 95
   Asp Ala Asp Gly Gln Asn Gly Leu Leu Gly Phe Ala Phe His Pro Asp
   100 105 110
   Phe Lys Asn Asn Pro Tyr Ile Tyr Val Ser Gly Thr Phe Lys Asn Pro
   115 120 125
   Lys Ser Thr Asp Lys Glu Leu Pro Asn Gln Thr Ile Ile Arg Arg Tyr
   130 135 140
   Thr Tyr Asn Lys Ala Thr Asp Thr Leu Asp Lys Pro Val Asp Leu Leu
   145 150 155 160
   Ala Gly Leu Pro Ser Ser Lys Asp His Gln Ser Gly Arg Leu Val Ile
   165 170 175
   Gly Pro Asp Gln Lys Ile Tyr Tyr Thr Ile Gly Asp Gln Gly Arg Asn
   180 185 190
   Gln Leu Ala Tyr Leu Phe Leu Pro Asn Gln Ala Gln His Thr Pro Thr
   195 200 205
   Gln Gln Glu Leu Ser Gly Lys Asp Tyr His Thr Tyr Met Gly Lys Val
   210 215 220
   Leu Arg Leu Asn Leu Asp Gly Ser Ile Pro Lys Asp Asn Pro Ser Phe
   225 230 235 240
   Asn Gly Val Ile Ser His Ile Tyr Thr Leu Gly His Arg Asn Pro Gln
   245 250 255
   Gly Leu Ala Phe Thr Pro Asn Gly Lys Leu Leu Gln Ser Glu Gln Gly
   260 265 270
   Pro Asn Ser Asp Asp Glu Ile Asn Leu Ile Val Lys Gly Gly Asn Tyr
   275 280 285
   Gly Trp Pro Asn Val Ala Gly Tyr Lys Asp Asp Ser Gly Tyr Ala Tyr
   290 295 300
   Ala Asn Tyr Ser Ala Ala Ser Asn Lys Ala Gln Ile Lys Asp Leu Gly
   305 310 315 320
   Gln Asn Gly Leu Lys Val Ala Ala Gly Val Pro Val Thr Lys Glu Ser
   325 330 335
   Glu Trp Thr Gly Lys Asn Phe Val Pro Pro Leu Lys Thr Leu Tyr Thr
   340 345 350
   Val Gln Asp Thr Tyr Asn Tyr Asn Asp Pro Thr Cys Gly Asp Met Thr
   355 360 365
   Tyr Ile Cys Trp Pro Thr Val Ala Pro Ser Ser Ala Tyr Val Tyr Lys
   370 375 380
   Gly Gly Lys Lys Ala Ile Ser Gly Trp Glu Asn Thr Leu Leu Val Pro
   385 390 395 400
   Ser Leu Lys Arg Gly Val Ile Phe Arg Ile Lys Leu Asp Pro Thr Tyr
   405 410 415
   Asn Thr Thr Tyr Asp Asp Ala Val Pro Met Phe Lys Ser Asn Asn Arg
   420 425 430
   Tyr Arg Asp Val Ile Ala Ser Pro Asp Gly Asn Val Leu Tyr Val Leu
   435 440 445
   Thr Asp Thr Ser Gly Asn Val Gln Lys Asp Asp Gly Ser Val Thr Asn
   450 455 460
   Thr Leu Glu Asn Pro Gly Ser Leu Ile Arg Phe Thr Tyr Lys Ala Gln
   465 470 475 480
<210> 25
   <211> 1443
   <212> DNA
   <213> Acinetobacter sp.
<400> 25
   atgaataaac atttattggc taaaattact ttattaggtg ctgctcagct agttacgctc      60
   aattcagcat ttgctgatgt ccctcttact ccatctcaat ttgctaaagc gaaaacagaa     120
   agctttgaca agaaagttct tatatctaat ttaaataagc cacatgcttt gttgtggggg     180
   cctgataatc aaatttggtt aacggagcgg gcaacaggta agattctaag agttaatcca     240
   gagtcgggca gtgtaaaaac agtttttcag gttcctgaga ttgtaaatga tgctgatgga     300
   caaaacggtt tattgggttt tgcctttcat cctgacttta aaaataatcc ttatatctat     360
   gtttcaggta catttaaaaa tccgaaatct acagataaag aattaccgaa tcaaactatt     420
   atccgtcgat atacctataa caaagcaaca gatactcttg agaaaccagt agatttattg     480
   gcaggattac cttcatcgaa agaccatcag tcgggtcgcc ttgtcattgg tccagaccaa     540
   aagatttact atacgattgg tgatcaggga cgtaaccagc ttgcttattt attcttacca     600
   aatcaggcac aacatacgcc gactcaacag gaactgagcg gaaaagacta tcatacctat     660
   atgggtaaag tattacgctt aaatctggat ggaagtattc caaaagataa tccaagcttt     720
   aacggtgtaa ttagtcatat ttatacgctc ggtcatcgta atccacaggg cttggcattt     780
   actccaaatg gtaaactgtt gcaatctgaa caaggtccaa actctgatga tgaaattaat     840
   ctcattgtta aaggtggcaa ctatggctgg ccaaatgtag cgggttataa agatgacagt     900
   ggttatgcct atgcaaatta ttcggcagca accaataagt cacaaattaa agatttaggg     960
   caaaatggag taaaagtagc ggcaggtgta cctgtgatga aagagtctga atggactggt    1020
   aaaaactttg taccgccgtt aaaaacttta tataccgtcc aagataccta taactataat    1080
   gacccaacct gtggggatat gacctacatt tgctggccaa cagttgcacc gtcatctgct    1140
   tatgtctata agggcggcaa aaaagcaatt tctggttggg aaaatacatt attggttcca    1200
   tctttaaagc gtggtgttat tttccgtatt aagctagatc caacttacag tgctacttat    1260
   gatgatgctg tgccgatgtt taagagcaac aatcgttatc gtgacgtgat tgcaagtcca    1320
   gatgggaatg ttttatatgt attgactgat acttccggaa atgtccaaaa agatgatggt    1380
   tctgtaacga atacattaga aaatccagga tctctgatta gatttacata taaagctcag    1440
   taa                                                                  1443
<210> 26
   <211> 480
   <212> PRT
   <213> Acinetobacter sp.
<400> 26
   Met Asn Lys His Leu Leu Ala Lys Ile Thr Leu Leu Gly Ala Ala Gln
   1               5                   10                  15
   Leu Val Thr Leu Asn Ser Ala Phe Ala Asp Val Pro Leu Thr Pro Ser
               20                  25                  30
   Gln Phe Ala Lys Ala Lys Thr Glu Ser Phe Asp Lys Lys Val Leu Ile
           35                  40                  45
   Ser Asn Leu Asn Lys Pro His Ala Leu Leu Trp Gly Pro Asp Asn Gln
       50                  55                  60
   Ile Trp Leu Thr Glu Arg Ala Thr Gly Lys Ile Leu Arg Val Asn Pro
   65                  70                  75                  80
   Glu Ser Gly Ser Val Lys Thr Val Phe Gln Val Pro Glu Ile Val Asn
                   85                  90                  95
   Asp Ala Asp Gly Gln Asn Gly Leu Leu Gly Phe Ala Phe His Pro Asp
               100                 105                 110
   Phe Lys Asn Asn Pro Tyr Ile Tyr Val Ser Gly Thr Phe Lys Asn Pro
           115                 120                 125
   Lys Ser Thr Asp Lys Glu Leu Pro Asn Gln Thr Ile Ile Arg Arg Tyr
       130                 135                 140
   Thr Tyr Asn Lys Ala Thr Asp Thr Leu Glu Lys Pro Val Asp Leu Leu
   145                 150                 155                 160
   Ala Gly Leu Pro Ser Ser Lys Asp His Gln Ser Gly Arg Leu Val Ile
                   165                 170                 175
   Gly Pro Asp Gln Lys Ile Tyr Tyr Thr Ile Gly Asp Gln Gly Arg Asn
               180                 185                 190
   Gln Leu Ala Tyr Leu Phe Leu Pro Asn Gln Ala Gln His Thr Pro Thr
           195                 200                 205
   Gln Gln Glu Leu Ser Gly Lys Asp Tyr His Thr Tyr Met Gly Lys Val
       210                 215                 220
   Leu Arg Leu Asn Leu Asp Gly Ser Ile Pro Lys Asp Asn Pro Ser Phe
   225 230 235 240
   Asn Gly Val Ile Ser His Ile Tyr Thr Leu Gly His Arg Asn Pro Gln
   245 250 255
   Gly Leu Ala Phe Thr Pro Asn Gly Lys Leu Leu Gln Ser Glu Gln Gly
   260 265 270
   Pro Asn Ser Asp Asp Glu Ile Asn Leu Ile Val Lys Gly Gly Asn Tyr
   275 280 285
   Gly Trp Pro Asn Val Ala Gly Tyr Lys Asp Asp Ser Gly Tyr Ala Tyr
   290 295 300
   Ala Asn Tyr Ser Ala Ala Thr Asn Lys Ser Gln Ile Lys Asp Leu Gly
   305 310 315 320
   Gln Asn Gly Val Lys Val Ala Ala Gly Val Pro Val Met Lys Glu Ser
   325 330 335
   Glu Trp Thr Gly Lys Asn Phe Val Pro Pro Leu Lys Thr Leu Tyr Thr
   340 345 350
   Val Gln Asp Thr Tyr Asn Tyr Asn Asp Pro Thr Cys Gly Asp Met Thr
   355 360 365
   Tyr Ile Cys Trp Pro Thr Val Ala Pro Ser Ser Ala Tyr Val Tyr Lys
   370 375 380
   Gly Gly Lys Lys Ala Ile Ser Gly Trp Glu Asn Thr Leu Leu Val Pro
   385 390 395 400
   Ser Leu Lys Arg Gly Val Ile Phe Arg Ile Lys Leu Asp Pro Thr Tyr
   405 410 415
   Ser Ala Thr Tyr Asp Asp Ala Val Pro Met Phe Lys Ser Asn Asn Arg
   420 425 430
   Tyr Arg Asp Val Ile Ala Ser Pro Asp Gly Asn Val Leu Tyr Val Leu
   435 440 445
   Thr Asp Thr Ser Gly Asn Val Gln Lys Asp Asp Gly Ser Val Thr Asn
   450 455 460
   Thr Leu Glu Asn Pro Gly Ser Leu Ile Arg Phe Thr Tyr Lys Ala Gln
   465 470 475 480
<210> 27
   <211> 1443
   <212> DNA
   <213> Acinetobacter sp.
<400> 27
   atgaataaac atttattggc taaaattact ttattaggtg ctgctcagct acttacgttc      60
   aattcagcat ttgctgatgt ccctcttact ccatctcaat ttgctaaagc gaaaacggaa     120
   agctttgata agaaagttct tctatctaat ttaaataagc cacatgcttt gttgtgggga     180
   gctgataatc aaatttggtt aacggagcgg gcaacaggta agattctaag agtgaatcca     240
   gagtcgggca gtgtaaaaac agtttttcag gttcctgaga ttgtaaatga tgctgatgga     300
   caaaacggtt tattgggttt tgcctttcat cctgacttta aaaataatcc ttatatctat     360
   gtttcaggta catttaaaaa tccgaaatct acagataaag aattaccgaa tcaaactatt     420
   atccgtcgat atacctataa caaggcaaca gatacccttg agaaaccagt agatttattg      480
   gcaggattac cttcatcgaa agaccatcag tcgggtcgcc ttgtcattgg tccagaccaa     540
   aaaatttact atacgattgg tgatcagggg cgtaaccagc ttgcttattt attcttgcca     600
   aatcaagcac agcatacgcc gactcaacag gaactgagcg gcaaagacta tcatacctat     660
   atgggtaaag tattacgctt aaatctggat ggaagtattc caaaagataa tccaagcttt     720
   aacggcgtaa ttagtcatat ttatacgctc ggtcatcgaa acccacaggg cttggcattt     780
   actccaaatg gtaaactgtt gcaatctgaa cagggtccaa actctgacga tgaaattaat     840
   ctcattgtta aaggtggtaa ctatggctgg ccaaatgtag cgggttataa agatgacagt     900
   ggttatgcct atgcaaatta ttcggcagca accaataagt cacaaattaa agatttaggg     960
   caaaatggag taaaagtagc ggcaggtgta cctgtgatga aagagtctga atggagtggt    1020
   aaaaactttg taccgccgtt aaaaacttta tataccgtcc aaggtaccta taactataat    1080
   gacccaacct gtggggatat gacctacatt tgctggccaa cggttgcgcc gtcatctgct    1140
   tatgtctata agggaggcaa aaaagcaatt tctggttggg aaaatacctt actggttcca    1200
   tctttaaagc gcggtgttat tttccgtatt aagctagatc caacttacag tgctacttat    1260
   gatgatgcgg tgccgatgtt taagagcaac aatcgttatc gtgacgtgat tgcaagtcca    1320
   gatgggaatg ttttatatgt attgactgat acttccggaa atgtccaaaa agatgatggt    1380
   tctgtaacga atacattaga aaacccagga tctctgatta gatttacata taaagctcag    1440
   taa                                                                  1443
<210> 28
   <211> 480
   <212> PRT
   <213> Acinetobacter sp.
<400> 28
   Met Asn Lys His Leu Leu Ala Lys Ile Thr Leu Leu Gly Ala Ala Gln
   1               5                   10                  15
   Leu Leu Thr Phe Asn Ser Ala Phe Ala Asp Val Pro Leu Thr Pro Ser
               20                  25                  30
   Gln Phe Ala Lys Ala Lys Thr Glu Ser Phe Asp Lys Lys Val Leu Leu
           35                  40                  45
   Ser Asn Leu Asn Lys Pro His Ala Leu Leu Trp Gly Ala Asp Asn Gln
       50                  55                  60
   Ile Trp Leu Thr Glu Arg Ala Thr Gly Lys Ile Leu Arg Val Asn Pro
   65                  70                  75                  80
   Glu Ser Gly Ser Val Lys Thr Val Phe Gln Val Pro Glu Ile Val Asn
                   85                  90                  95
   Asp Ala Asp Gly Gln Asn Gly Leu Leu Gly Phe Ala Phe His Pro Asp
               100                 105                 110
   Phe Lys Asn Asn Pro Tyr Ile Tyr Val Ser Gly Thr Phe Lys Asn Pro
           115                 120                 125
   Lys Ser Thr Asp Lys Glu Leu Pro Asn Gln Thr Ile Ile Arg Arg Tyr
       130                 135                 140
   Thr Tyr Asn Lys Ala Thr Asp Thr Leu Glu Lys Pro Val Asp Leu Leu
   145                 150                 155                 160
   Ala Gly Leu Pro Ser Ser Lys Asp His Gln Ser Gly Arg Leu Val Ile
                   165                 170                 175
   Gly Pro Asp Gln Lys Ile Tyr Tyr Thr Ile Gly Asp Gln Gly Arg Asn
               180                 185                 190
   Gln Leu Ala Tyr Leu Phe Leu Pro Asn Gln Ala Gln His Thr Pro Thr
           195                 200                 205
   Gln Gln Glu Leu Ser Gly Lys Asp Tyr His Thr Tyr Met Gly Lys Val
       210                 215                 220
   Leu Arg Leu Asn Leu Asp Gly Ser Ile Pro Lys Asp Asn Pro Ser Phe
   225                 230                 235                 240
   Asn Gly Val Ile Ser His Ile Tyr Thr Leu Gly His Arg Asn Pro Gln
                   245                 250                 255
   Gly Leu Ala Phe Thr Pro Asn Gly Lys Leu Leu Gln Ser Glu Gln Gly
               260                 265                 270
   Pro Asn Ser Asp Asp Glu Ile Asn Leu Ile Val Lys Gly Gly Asn Tyr
           275                 280                 285
   Gly Trp Pro Asn Val Ala Gly Tyr Lys Asp Asp Ser Gly Tyr Ala Tyr
       290                 295                 300
   Ala Asn Tyr Ser Ala Ala Thr Asn Lys Ser Gln Ile Lys Asp Leu Gly
   305                 310                 315                 320
   Gln Asn Gly Val Lys Val Ala Ala Gly Val Pro Val Met Lys Glu Ser
                   325                 330                 335
   Glu Trp Ser Gly Lys Asn Phe Val Pro Pro Leu Lys Thr Leu Tyr Thr
               340                 345                 350
   Val Gln Gly Thr Tyr Asn Tyr Asn Asp Pro Thr Cys Gly Asp Met Thr
           355                 360                 365
   Tyr Ile Cys Trp Pro Thr Val Ala Pro Ser Ser Ala Tyr Val Tyr Lys
       370                 375                 380
   Gly Gly Lys Lys Ala Ile Ser Gly Trp Glu Asn Thr Leu Leu Val Pro
   385                 390                 395                 400
   Ser Leu Lys Arg Gly Val Ile Phe Arg Ile Lys Leu Asp Pro Thr Tyr
                   405                 410                 415
   Ser Ala Thr Tyr Asp Asp Ala Val Pro Met Phe Lys Ser Asn Asn Arg
               420                 425                 430
   Tyr Arg Asp Val Ile Ala Ser Pro Asp Gly Asn Val Leu Tyr Val Leu
           435                 440                 445
   Thr Asp Thr Ser Gly Asn Val Gln Lys Asp Asp Gly Ser Val Thr Asn
   450 455 460
   Thr Leu Glu Asn Pro Gly Ser Leu Ile Arg Phe Thr Tyr Lys Ala Gln
   465 470 475 480

## Beschreibung

Die Erfindung betrifft neuartige Pyrrolochinolinchinon- bzw. Pyrroloqinolinquinon (PQQ) abhängige lösliche Glukose Dehydrogenasen (sPQQGDH) aus *Acinetobacter* sowie ein Verfahren zu deren Herstellung durch Überexpression in geeigneten mikrobiellen Expressionssystemen.

Patienten, die unter Diabetes leiden, müssen ihren Blutzucker regelmäßig messen. Ebenso spielt die Messung von Glukose bei Fermentationsprozessen eine wichtige Rolle. Vielfach erfolgt die Bestimmung von Glukose enzymatisch. Dazu werden entweder Glukose Oxidase oder in seltenen Fällen auch Glukose-6-phosphat Dehydrogenase eingesetzt. Die Verfahren, die auf Glukose Oxidase beruhen, haben den Nachteil, dass das Enzym die Elektronen auch auf den anwesenden Sauerstoff und nicht ausschließlich auf die zugesetzten Mediatoren überträgt. Daher ist das Ergebnis der Messung vom Partialdruck des Sauerstoffs abhängig. Seit geraumer Zeit ist man darum bemüht, vorhandene Enzyme durch sPQQ abhängige Glucosedehydrogenasen zu ersetzen. PQQ steht für Pyrrolochinolinchinon oder Pyrroloqinolinquinon. PQQ ist die prosthetische Gruppe der Glucosedehydrogenase (GDH). Sie überträgt Redoxäquivalente. Der Vorteil dieser Enzyme liegt darin, dass der Messwert vom Partialdruck des Sauerstoffs unabhängig ist und eine genauere Messung möglich wird. Weiter wird durch den Einsatz dieser Enzyme die Messung in kleineren Probenvolumen möglich.

In der Literatur findet man zwei verschiedene PQQGDHs. Die eine Form ist membrangebunden (mPQQGDH) und für den Einsatz in Glukosesensoren ungeeignet. Die andere From ist löslich (sPQQGDH) und ist verschiedentlich in Stämmen aus der Gattung *Acinetobacter* gefunden worden (Biosci. Biotech. Biochem. 59 (8), S. 1548-1555, 1995). Das Enzym ist ein Homodimer und besitzt ein Molekulargewicht von 50 kDa. Die lösliche und die membrangebundene PQQGDH besitzen keine Sequenzhomologie und sind immunologisch und bezüglich ihrer Kinetik unterschiedlich (Cleton-Jansen et al., 172 (11), S. 6308 - 6315, J. Bacteriol. 1990) (Matsushita et al., Biochemistry 28 (15), S. 6276 - 6280, 1989).

Die sPQQGDH aus *Acinetobacter* ist seit längerem bekannt. Alle Autoren arbeiten mit den Stämmen LMD 79.41 (Kojima et al., Biotechnology Letters, 22, S. 1343 - 1347, 2000), NCIMB 11517 oder JCM 6841 (beide: US 2001021523).

Die DNA Sequenzen der sPQQGDH dieser Stämme sind in der Genbank unter den Zugriffsnummern X15871 (LMD 79.41), E28183 (JCM 6841) und E28182 (NCIMB 11517) hinterlegt.

Es ist vielfach versucht worden, die Eigenschaften dieser sPQQGDH durch Modifikation der Gensequenzen zu verändern. (EP1 167 519 A1, EP 1 176 202 A1, Sode und Kojima, Biotechn. Letters, Vol. 19, (11) S. 1073-1077, 1997). Ziel war es dabei, die Substratspezifität und die Thermostabilität des Enzyms zu verbessern. EP 1 167 519 A1 beschreibt den Austausch einzelner Aminosäuren der sPQQGDH aus *Acinetobacter* LMD 79.41, um eine erhöhte Thermostabilität zu erhalten. Veränderungen werden an den Aminosäuren 209, 210, 231, 420 und 421 vorgenommen. WO 02/072839 A1 beschreibt weitere Veränderungen an der Aminosäuresequenz, um erhöhte Thermostabilitäten und Verbesserungen in der Wasserlöslichkeit zu erreichen. Es wurden die Positionen 167, 231, 340, 415 und 418 ausgetauscht. Die Zählweise beruht in beiden Fällen auf der in der EP 1 167 519 verwendeten. Bei Vergleichen des Enzyms aus *Acinetobacter* LMD 79.41 mit den erfindungsgemäßen neuen sPQQGDH sowie der aus *Acinetobacter* JCM 6841 muß weiter berücksichtigt werden, dass die beiden letztgenannten Enzyme um zwei Aminosäuren länger sind als das aus LMD 79.41. Betrachtet wird hierbei das reife Enzym und das Signalpeptid.

Die WO 02/34919 A1 beschreibt den Austausch einzelner Aminosäuren mit dem Ziel, die Affinität des Enzyms für Maltose zu verringern. Die Thermostabilität des Enzyms wird nicht beeinflusst.

Zur Herstellung von Enzymen werden diese üblicherweise in *E. coli* heterolog exprimiert. Bei der Expression von sPQQGDH entsteht nun das Problem, dass *E. coli* zwar das Enzym, nicht aber den Kofaktor PQQ synthetisieren kann. Zwar gelingt laut Sode et al. (J. Biotechnology, 49, 239 - 243, 1996) im Fall der membrangebundenen Glucosedehydrogenase die Expression des Apoenzyms mPQQGDH, dieses ist jedoch nicht stabil und wird während der Zellanzucht wieder abgebaut. Matsushita et al. (Biosci. Biotechnol. Biochem. 59, 1548 - 1555, 1995) postulieren eine Konformationsänderung des Enzyms, die bei der Bindung des Kofaktors eintritt und das Holoenzym gegen tryptischen Verdau schützt.

Daher haben alle Arbeitsgruppen und Hersteller sich darum bemüht, aktives und stabiles Enzym so herzustellen, dass entweder PQQ während der Zellanzucht zugesetzt wird oder die Expression in Stämmen erfolgt, die selber PQQ bilden können. Sode et al. (J. Biotechnology, 49, 239 - 243, 1996) gelang die Koexpression der mPQQGDH gemeinsam mit den Genen für die PQQ Synthese in *E. coli* und erreichte bei einer OD von 4 etwa 1.500 U/l. Wird das PQQ während der Zellanzucht zugesetzt, so werden 1.100 U/l erreicht. Allerdings hat das System das Problem, dass die Zellen keine hohen optischen Dichten erreichen und während der Enzymproduktion absterben (Kojima et al., Biotechnology Letters, 22, 1343 - 1347, 2000). Die Autoren beschreiben in der zuvor zitierten Publikation die Synthese aktiver sPQQGDH in *Klebsiella pneumoniae.* Dieser Organismus ist genetisch zugänglich und besitzt die Fähigkeit, PQQ zu synthetisieren. Daher werden dabei Aktivitäten von etwa 14.000 U/l erreicht, allerdings muss zusätzliches PQQ zugefüttert werden. Yoshida et al. (Enzym. Microb. Technol. 30, S. 312 - 318, 2002) haben die lösliche sPQQGDH in *Pichia pastoris* heterolog exprimiert und erreichen damit brauchbare Enzymausbeuten von bis zu 200.000 U/l. Allerdings müssen dazu sehr hohe Zelldichten erreicht werden. Daneben ist das Enzym glykosyliert und muß zudem über eine Fällung und zwei Ionenaustauscher gereinigt werden. Insbesondere die aufwendige Reinigung und die Glykosylierung erschweren eine kostengünstige Herstellung.

Die Firma Toyobo Co. Ltd., Japan beschreibt in JP 09140378 die Herstellung von sPQQGDH mit *Acinetobacter calcoaceticus.* Nach drei Reinigungsschritten werden 60 U / l erreicht. Um die Produktivität des Systems zu erhöhen, wurde das Enzym anschließend heterolog in *Pseudomonas putida* exprimiert, da dieser Stamm in der Lage ist, PQQ zu synthetisieren.

Olsthoorn und Duine (Arch. Biochem. Biophys. 336 (1), 42 - 48, 1996) beschreiben die Batch-Anzucht eines *E. coli*-Klones, der eine sPQQGDH exprimiert, im 100-1-Fermenter. Nach der Anzucht wird das Enzym über drei Säulenschritte gereinigt. Der Klon bildet kein PQQ, der Kofaktor wird erst beim Enzymtest zugesetzt. Die Ausbeuten betragen 10 mg reines Protein pro 1 ausgewachsene Kultur. Die geringe Zelldichte der Kultur, das aufwendige Reinigungsverfahren und die Ausbeute lassen das Verfahren aber unwirtschaftlich erscheinen.

### Beschreibung der Erfindung

Im Rahmen eines Screenings wurden 12 Stämme der Art *Acinetobacter calcoaceticus* gefunden, die eine sPQQGDH bilden. Eine Typisierung der neuen Stämme durch die Deutsche Sammlung von Mikroorganismen und Zellkulturen (DSMZ) ergab eine 99,8 %ige Homologie der partiellen 16S rDNA Sequenz zum Typstamm *Acinetobacter calcoaceticus.* Allerdings ist die beobachtete Verwertung von L-Malat und die Unfähigkeit, L-Phenylacetat abzubauen, für den Typstamm ungewöhnlich. Alle 12 gefundenen Stämmen zeigen diese Eigenschaft.

Die von diesen Stämmen gebildeten sPQQGDHs unterscheiden sich von den bisher bekannten Enzymen in wesentlichen Eigenschaften. Zum einen besitzen sie eine gegenüber dem Enzym aus dem Typstamm verbesserte Thermostabilität (siehe Beispiel 3). Die Nukleotidsequenzen sowie die Sequenz der Aminosäuren unterscheiden sich ebenfalls.

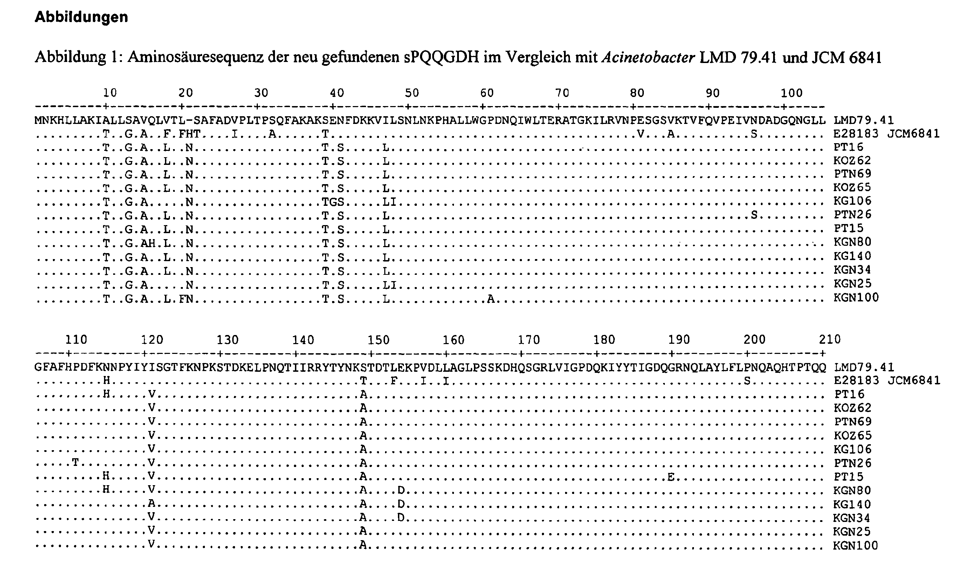

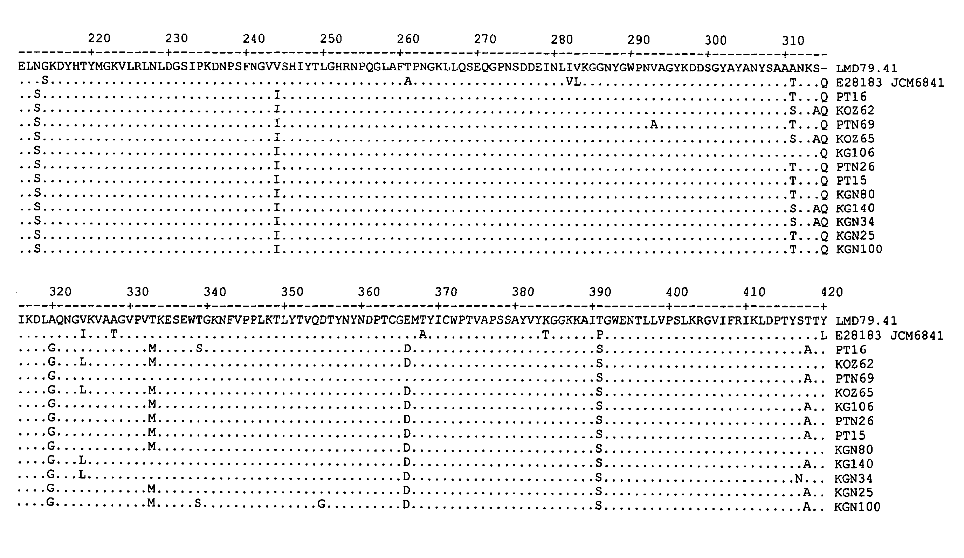

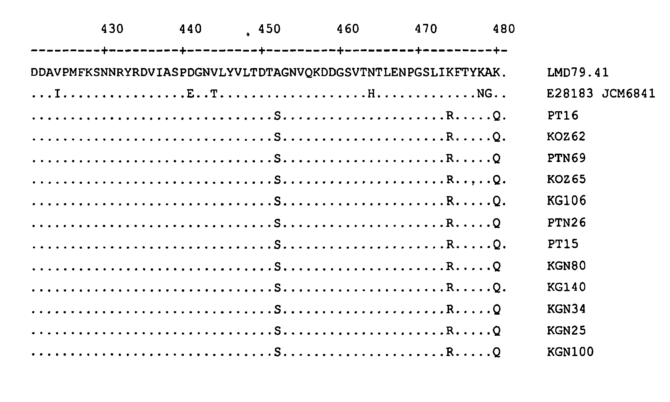

Die Sequenzanalyse der 12 Gene, die für die neuartigen sPQQGDH kodieren, ergab folgende Ergebnisse (siehe Abbildung 1):
Die Nukleotidsequenzen aller neu gefundenen sPQQGDH unterschieden sich deutlich von denen der Enzyme aus *Acinetobacter* LMD 79.41 und *Acinetobacter* JCM 6841 sowie NCIMB 11517.
Die Aminosäuresequenzen, die sich aus den jeweiligen Nukleotidsequenzen ableiten lassen, unterscheiden sich ebenfalls von den bisher bekannten Sequenzen. Die sPQQGDH aus *Acinetobacter* LMD 79.41 hat einschließlich des Signalpeptids 478 Aminosäuren, die von den beiden anderen bekannten Stämmen *Acinetobacter* JCM 6841 und *Acinetobacter* NCIMB 11517 sowie die von den hier gefundenen Sequenzen haben einschließlich Signalpeptid je 480 Aminosäuren.
Die Aminosäuresequenzen der neu gefundenen sPQQGDH unterscheiden sich an zahlreichen Positionen von den bisher bekannten. Überraschenderweise wurde an 12 Positionen bei allen hier neu beschriebenen Genen eine andere Aminosäure als in allen vorbeschriebenen Enzymen gefunden. Zusätzlich tragen mindestens 75 % der hier neu beschriebenen Gene an vier Positionen eine andere Aminosäure als die vorbeschriebenen Gene. Darüber hinaus gibt es bei den hier neu beschriebenen Enzymen weitere Austausche, die vereinzelt oder bei einem Teil der Enzyme vorkommen.

Im einzelnen sind folgende Aminosäuren der neu gefundenen erfindungsgemäßen sPQQGDH ausgetauscht. Die Zählung der Aminosäuren orientiert sich an der Zählung im Enzym aus dem Stamm *Acinetobacter* JCM 6841, das einschließlich Signalpeptid 480 Aminosäuren umfasst (siehe Abbildung 1):
Erfindungsgemäß sind alle die löslichen Pyrrolochinolinchinon- bzw. Pyrroloqinolinquinon Glucose Dehydrogenase (sPQQGDH), die an den Positionen 21 ein N und 41 ein S und 47 ein L und 121 ein V oder ein A und 149 ein A und 213 ein S und 244 ein I und 320 ein G und 391 ein S und 452 ein S und 474 ein R und 480 ein Q als Aminosäure haben.

Weiter sind alle die sPQQGDH erfindungsgemäß, bei denen zusätzlich zu den oben beschrieben Austauschen folgende Austausche vorkommen können:
An der Positionen 16 kann ein H, an 18 ein L, an 20 ein F, an 40 ein G, an 48 ein I, an 61 ein A, an 111 ein T, an 154 ein D, an 190 ein D, an 293 ein A, an 311 ein S, an 314 ein A, an 324 ein L, an 333 ein M, an 339 ein S, an 355 ein G, an 366 ein D, an 417 ein N und an 418 ein A stehen.

Es wurde überraschenderweise gefunden, dass die neu gefundenen Enzyme alle thermostabiler sind als das Wildtypenzym. Die Thermostabilität ist ein wesentliches Kriterium für den erfolgreichen Einsatz von sPQQGDH als Glukosesensor. So wurden bei einer einstündigen Inkubation bei 60°C und 70°C durchweg höhere Restaktivitäten gefunden als beim Enzym aus *Acinetobacter* LMD 79.41. Bei 60°C wurden bei den erfindungsgemäßen sPQQGDH zwischen 51,5 % und 12,5 % Restaktivität gefunden. Der Wildtyp wies in einem identischen Ansatz lediglich 1,8 % seiner ursprünglichen Aktivität auf. Bei 70°C wurden zwischen 26,7 % und 8,8 % der Ausgangsaktivität beobachtet, das Enzym aus dem Wildtyp besaß hingegen lediglich 6,4 % der ursprünglichen Aktivität.

In der Vergangenheit ist verschiedentlich versucht worden, die Thermostabilität von sPQQGDH durch gezielten und zufälligen Austausch von Aminosäuren zu erhöhen.

Die EP 1 167 519 A1 beansprucht allerdings den Austausch von Aminosäuren an Positionen, die sich von denen in den erfindungsgemäßen sPQQGDH deutlich unterscheiden. Das gleiche gilt für die Austausche, die in WO 02/072839 offenbart sind. Ebenso gilt dies für die Austausche, die in WO 02/34919 A1 beschrieben sind. Die erhöhte Thermostabilität der erfindungsgemäßen sPQQGDH korreliert somit mit dem Austausch einer ganzen Gruppe von Aminosäuren, deren Einfluss auf diese Eigenschaft bisher nicht bekannt war.

Zur Herstellung der erfindungsgemäßen Enzyme kann der betreffende Stamm in einem geeigneten Medium kultiviert werden. Hierzu können die in der Literatur beschriebenen Medien wie Nutrient broth (Difco 0003) benutzt werden. Nach Auswachsen der Zellen werden diese geerntet und aufgeschlossen. Die Reinigung des Enzyms erfolgt wie unten beschrieben.

Bevorzugt werden die sPQQGDHs aus dem Wildtyp in einen geeigneten Wirt kloniert. Dies können die üblichen genetisch gut zugänglichen Prokaryonten wie Angehörige der Gattung *Bacillus, Klebsiella, Pseudomonas* sowie *E. coli* sein. Wieter können die Enzyme auch in Eukaryonten wie Angehörige der Gattungen *Pichia, Saccharomyces, Hansenula, Aspergillus* oder *Kluyveromyces* exprimiert werden. Die Enzyme können auch in Pflanzen oder tierischen Zelllinien exprimiert werden.

Zur Klonierung können Standardmethoden wie PCR mit degenerierten Primern oder Hybridisierung von genomischen Bibliotheken mit geeigneten Sonden eingesetzt werden. Bevorzugt wird die Expression in *E. coli* durchgeführt. Die Expression wird üblicherweise in den Stämmen BL21, DH5, HB101, JM101, RV308, TOPP, TOP10, XL-1 sowie deren Derivaten durchgeführt. Bevorzugt werden die Stämme W3110 und DH5 benutzt. Dazu können die gängigen Expressionsvektoren eingesetzt werden. Die Vektoren enthalten typischerweise einen Replikationsursprung, eine Resistenz gegen ein Antibiotikum und eine Promotersequenz. Folgende Vektoren können z.B. eingesetzt werden pUC18/19, pBluescript, pTZ, pGEX, pPROEx, pcDNA3.1, YEp24, pBAC, pPICZ. Bevorzugt werden die Vektoren der Serien pMAL, pET, pTrx, pCAL, pQE und pPROTet. Besonders bevorzugt werden die Expressionsvektoren der Serie pASK-IBA 2 bis pASK-IBA 7 eingesetzt. Übliche Antibiotika, auf deren Resistenz selektiert wird, sind z.B. Ampicillin, Kanamycin, Tetrazyklin und Chloramphenicol.

Es können auch Expressionssysteme benutzt werden, die dazu führen, dass das Protein ins Medium ausgeschieden wird.

Die Vektoren können mit den üblichen Verfahren auf die Wirtszelle übertragen werden. Dazu werden beispielsweise eingesetzt: Elektroporation, Protoplastenfusion, chemische Transformation.

Die Synthese der klonierten sPQQGDH wird durch Zusatz eines Induktors induziert. Hierzu werden die für das gewählte Expressionssystem geeigneten Induktoren wie z.B. IPTG, Tryptophan, Glukose und Laktose eingesetzt. Besonders bevorzugt wird der Induktor Anhydrotetrazyklin verwandt.

Die rekombinanten Zellinien werden in für sie geeigneten Medien angezogen. Dazu sind die gängigen Verfahren zur Anzucht von Pro- und Eukaryonten geeignet. Die Anzucht kann in geeigneten Fermentern durchgeführt werden. Bevorzugt werden die Organismen so kultiviert, das sehr hohe Zelldichten erreicht werden. Dazu ist es erforderlich, dass mit einer geeigneten Regelstrategie die Zufütterung einer C-Quelle und einer N-Quelle so erfolgt, dass keine toxischen Stoffwechselprodukte entstehen (zur Übersicht: Schügerl et al. (Hrsg) in: Bioreaction Engineering, S. 374-390, Springer-Verlag, Berlin, 2000; Yee und Blanch, Bio/Technology, 10 (2), S. 1550 - 1556, 1992).

Ein im Sinne dieser Erfindung geeignetes Verfahren ist beispielsweise das nach Riesenberg et al., Appl. Microbiol. Biotechnol, 34, S. 77 - 82, 1990.

Während die Kultivierung der Zellen idealerweise bei 28 - 37°C erfolgt wird für die Proteinexpression die Temperatur auf 10 - 28°C gesenkt. Bevorzugt liegt sie bei 15 - 25°C und besonders bevorzugt bei 20 - 22°C.

Überraschenderweise wurde gefunden, dass bei Verwendung des erfindungsgemäßen Verfahrens deutlich höhere Ausbeuten an aktivem Enzym erreicht werden als bisher in der Literatur beschrieben. Bei einer Anzucht eines E. coli-Klons in Batch-Kultur können auf diese Weise bis zu 48 mg reines Protein aus einem Liter Kulturüberstand erhalten werden (siehe Beispiele 5 und 6). Yoshida et al. (Enzym. Microbiol. Technol., 30, S. 312 - 318, 2002) erreichen 43 mg / 1. Allerdings muss das Enzym aufwendig gereinigt werden und ist glykosyliert. Olsthoorne und Duine (Arch. Biochem. Biophys. 336 (1), S. 42 - 48, 1996 berichten über eine Ausbeute von 10 mg / L.

Werden die Zellen mit den erfindungsgemäßen Enzymen in einer Hochzelldichte Fermentation angezogen, so können sogar über 220 mg reines Enzym pro L Kulturflüssigkeit erreicht werden (Beispiel 8).

Nach der Anzucht der Zellen werden diese geerntet und mit geeigneten Methoden wie z.B. French Presse, Zusatz von Detergentien oder Ultraschall aufgeschlossen. Die Proteinlösung wird gepuffert und auf einen leicht alkalischen pH-Wert gebracht. Der Puffer wird auf einen pH-Wert zwischen 7 und 9 eingestellt, bevorzugt liegt er zwischen 7,8 und 8,2.

Als Puffersubstanzen können die in der Biochemie üblichen Puffer wie Tris-, MOPS- oder PIPES-Puffer, Kaliumphosphat-Puffer eingesetzt werden, die Konzentration sollte bei 5-100 mM liegen, bevorzugt liegt sie in einem Bereich von 20 - 70 mM und besonders bevorzugt bei 50 mM.

Die sPQQGDH kann nun sehr einfach aus der Proteinlösung aufgereinigt werden. Dazu wird die Proteinlösung entweder durch eine konventionelle Ionenaustauschchromatographie gereinigt oder das Ionenaustauschermaterial wird der Proteinlösung direkt zugesetzt und anschließend über eine Nutsche vom Rest der Flüssigkeit getrennt. Als Ionenaustauscher kommen Kationenaustauscher wie z.B. Lewatitharze, CM-, S-, SM-Sepharose, CM-, SP-Sephadex, Amberlyst 15, Amberlite CG-50, Amberlite IR-120, Carboxymethyl-, Sulfoxyethyl-, Oxycellulose, Cellulosephosphat und CM-Toyopearl in Frage. Bevorzugt wird CM-Toyopearl eingesetzt.

Das Protein wird anschließend mit den üblichen Methoden vom Ionenaustauschermaterial eluiert. Dazu wird ein steigender NaCl-Gradient eingesetzt, es wird der Puffer verwandt, der auch zuvor beim Binden des Proteins an das Ionenaustauschermaterial eingesetzt wurde. Üblicherweise euliert das Enzym bei einer Konzentration von ca. 200 mM NaCl.

Das Enzym kann dann noch weiter gereinigt werden, ebenso kann durch die üblichen Methoden wie Dialyse und Ultrafiltration der Gehalt an Salz reduziert werden.

Die Herstellung und Aufreinigung der erfindungsgemäßen sPQQGDH erfolgt bevorzugt als Apoenzym, der Cofaktor PQQ wird erst zugesetzt, wenn das Enzym vollständig gereinigt wurde. Idealerweise erfolgt die Zugabe des PQQ im Zusammenhang mit der Umpufferung des Enzyms nach der Reinigung über den Ionenaustauscher.

Das PQQ kann vor der Umpufferung oder danach zugesetzt werden. Bevorzugt wird es davor zugesetzt. Die Menge richtet sich nach dem Gehalt der Proteinlösung. Pro Mol aktivem Enzym kann 0,1 bis 5 mol PQQ zugesetzt werden. Idealerweise werden 0,5 bis 2 Mol, besonders bevorzugt 2 mol PQQ pro Mol aktivem Protein zugesetzt. Es ist aber auch möglich, das Enzym als Holoenzym herzustellen und zu reinigen. Dazu kann PQQ während der Zellanzucht, beim Zellaufschluss oder vor der Reinigung zugesetzt werden. Weiter können die erfindungsgemäßen sPQQGDH auch als Holoenzyme hergestellt werden, indem sie in Organismen heterolog exprimiert werden, die PQQ synthetisieren können. Dies können beispielsweise Organismen aus der Gattung *Klebsiella* oder *Pseudomonas* sein.

Die neuartigen sPQQGDH werden erfindungsgemäß zur Messung von Glukose eingesetzt. Besonders bevorzugt werden sie in Geräten eingesetzt, mit denen Blutglukose gemessen werden kann. Daneben ist es auch möglich, die Enzyme zur Messung von Glukose beispielsweise in Fermentationsprozessen einzusetzen.

Werden die erfindungsgemäßen sPQQGDH zu diagnostischen Zwecken eingesetzt, so umfasst ein solcher Testkit typischerweise einen Puffer, einen Mediator und einige units Enzymaktivität. Typischerweise werden 0,5 - 10 U eingesetzt und bevorzugt werden 1 - 5 U eingesetzt. Das Enzym kann dafür unterschiedlich formuliert werden. Es kann beispielsweise gefrier- oder sprühgetrocknet sowie als Lösung formuliert sein. Geeignete Elektroden können Kohle-, Gold- oder Platinelektroden sein. Üblicherweise wird das Enzym auf der Elektrode immobilisiert. Üblicherweise werden dazu quervernetzende Agentien benutzt, das Enzym kann aber auch verkapselt sein. Weiter kann es mittels einer Dialysemembran, durch photo vernetzende, elektrisch leitende oder redox polymere immobilisiert werden. Auch Kombinationen der oben genannten Methoden sind möglich.

Bevorzugt werden die Enzyme als Holoenzyme aufgebracht, sie können aber auch als Apoenzyme eingesetzt werden wobei das erforderliche PQQ über eine zweite Schicht zugeführt wird. Bevorzugt werden die neuartigen sPQQGDH auf einer Kohlenstoffelektrode mit Glutaraldehyd immobilisiert und anschließend mit einem Amine enthaltenden Reagenz behandelt, damit überschüssiges Glutaraldehyd abreagiert.

### Beispiele

### Beispiel 1: Suche nach neuartigen Stämmen, die sPQQGDH bilden

Bodenproben wurden in Saline (0,9 % NaCl) suspendiert und Aliquots wurden auf Agarplatten ausgestrichen, die einen Nährboden mit Gluconat als einziger Kohlenstoffquelle enthielten. Das Medium war wie folgt zusammengesetzt:

| | |
|---|---|
| NaCl | 5 g |
| MgSO₄ | 0,2 g |
| NH₄H₂P0₄ | 1 g |
| K₂HPO₄ | 1 g |
| Natriumgluconate | 2 g |
| Hefeextrakt | 0,5 g |
| Agar | 15 g |
| Aqua dest. | 1000 ml |
| pH | 7,0 |

Die Platten wurden bei 30°C für 24 - 48 Stunden inkubiert.

Alternativ wurde auch *Pseudomonas*-Agar von Oxoid verwendet. Die Inkubationsbedingungen waren identisch.

Ausgewählte gewachsene Kolonien wurden auf Glukose-Eosin-Methylenblau-Agar vereinzelt. Dieser war wie folgt zusammengesetzt:

| | |
|---|---|
| K₂HP0₄ | 1 g |
| Glukose | 18 g |
| Peptone | 10 g |
| Eosine | 0,4 g |
| Methylenblau | 0,06 g |
| Agar | 16 g |
| Aqua dest. | 1000 ml |
| pH | 7,6 |

Die Platten wurden bei 30°C für 24 Stunden inkubiert. Positive Klone kann man daran erkennen, dass sie dunkelrot sind und/oder einen grünen Glanz besitzen. Diese Kolonien wurden erneut auf Nutrient Agar (Oxoid) ausgestrichen um sie auf Reinheit zu überprüfen. Dieser war wie folgt zusammengesetzt:

| | |
|---|---|
| Fleischextrakt | 1 g |
| Hefeextrakt | 2 g |
| Peptone | 5 g |
| NaCl | 5 g |
| Agar | 15 g |
| Aqua dest. | 1000 ml |
| pH | 7,4 |

Die Platten wurden für 48 Stunden bei 30°C inkubiert und erneut vereinzelt. Die gereinigten Stämme wurden anschließend für 20 h bei 30°C in 100 ml flüssigem Nutrient broth (Oxoid) angezogen, das Medium hatte folgende Zusammensetzung:

| | |
|---|---|
| Fleischextrakt | 3 g |
| Baktopepton | 5 g |
| Glukose | 1 g |
| Aqua dest. | 1000 ml |

Nach der Anzucht wurden die Kulturen geerntet (4.500 x g, 40 min, 4°C) und mit Saline (0,9 % NaCl) gewaschen. Die Pellets wurden in 5 ml 50 mM KP-Puffer pH 7,2 resuspendiert und mit Ultraschall aufgeschlossen. Der Extrakt wurde erneut für 30 min bei 4°C mit 10.000 x g zentrifugiert, im zellfreien Überstand wurde die GDH Aktivität gemessen. Auf dem Screening Medium mit Glukonat als einziger Kohlenstoffquelle konnten 26 Stämme mit GDH-Aktivität isoliert werden. Vier Stämme konnten nach Anzucht auf dem *Pseudomonas* Medium isoliert werden.

### Beispiel 2: Reinigung der Enzyme

Die Stämme, deren sPQQGDH untersucht werden sollten, wurden in 8 1 NB-Medium (Oxoid), das 0,1 % Glukose enthielt, für 20 h bei 30°C angezogen. Die Zellen wurden geerntet (4500 x g, 40 min, 4°C), mit 0,9 % Saline gewaschen und 150 - 200 ml 10 mM MOPS pH 8,0 aufgenommen. Der Zellaufschluss erfolgte mit Ultraschall. Der zellfreie Extrakt wurde auf 300 ml TSKgel CM-Toyopearl 650M (Tosoh Corp.) aufgetragen und mit 3-4 Säulenvolumen 10 mM MOPS pH 8,0 gewaschen. Die Elution erfolgte mit einem Gradienten 0 - 0,3 M NaCl mit einem 3 - 4fachen Volumen der Säule. Aktive Fraktionen wurden gepoolt, in einen Dialyseschlauch überführt und durch Zusatz von hoch viskoser Carboxymethylcellulose als Wasserabsorber konzentriert. Die konzentrierte Probe wurde mit 3,5 Volumen 10 mM K-MOPS pH 6,8 resuspendiert und erneut über die CM-Toyopearl Säule gereinigt. Die Äquilibirierung der Säule und das Waschen erfolgte mit 10 mM K-MOPS pH 6,8, die Elution wurde mit einem 0 - 0,3 M NaCl - Gradient durchgeführt. Die aktiven Fraktionen wurde gepoolt und wie oben beschrieben eingeengt. Sie dienten als Ausgangsmaterial für die Bestimmung der Thermostabilität.

### Beispiel 3: Test auf Thermostabilität

Die gereinigten Enzyme wurden zur Bestimmung der Thermostabilität für 60 Minuten bei 50, 60 und 70°C inkubiert. Die Inkubation erfolgte in 50 mM Pipes bei pH 6.5 in Gegenwart von 1 mM CaCl₂, 0,1% Triton X-100, 0,1 % BSA und 5 µM PQQ. Nach der Inkubation wurden die Proben auf Eis gekühlt und die Restaktivität wurde bestimmt. Sie wurde in Prozent der ursprünglichen Aktivität ausgedrückt.

**Tabelle 1: Thermostabilität neuartiger sPQQGDH aus verschiedenen Neuisolaten**

| **Stamm** | **50°C** | **60°C** | **70°C** |
|---|---|---|---|
| KGN25 | 100 | 14.3 | 13.1 |
| KGN34 | 104.2 | 19.8 | 14.6 |
| KGN80 | 94.3 | 22.6 | 18.9 |
| KGN 100 | 95.9 | 12.5 | 13.5 |
| KG 106 | 102.6 | 33.4 | 13.9 |
| KG 140 | 105.5 | 20.5 | 8.8 |
| KOZ62 | 107.7 | 42.8 | 14.7 |
| KOZ65 | 103.8 | 34.6 | 19.2 |
| PT15 | 100.0 | 25.4 | 16.3 |
| PT16 | 85.0 | 51.5 | 14.9 |
| PTN26 | 120.7 | 27.5 | 22.4 |
| PTN69 | 93.2 | 36.4 | 26.7 |
| LMD79.41 Wildtyp | 95.6 | 1.8 | 6.4 |

### Beispiel 4: Klonierung und Analyse der neuartigen sPQQGDH-Gene

Zur Klonierung der sPQQGDH-Gene aus den hier beschriebenen neuen Stämmen wurde zunächst versucht, in einer PCR auf genomischer DNA mit synthetischen Oligonukleotiden die vollständige kodierende Sequenz zu amplifizieren. Die dazu verwendeten, aus der publizierten sPQQGDH-Sequenz (X15871; LMD 79.41) abgeleiteten Primer führten allerdings nicht zu PCR-Produkten, da sich die Sequenzen der erfindungsgemäßen sPQQGDHs im Bereich des Signalpeptids und nahe des Stopcodons besonders stark vom Wildtyp unterscheiden. Deshalb wurden verschiedene Primer aus beiden Strängen der Wildtyp-Sequenz verwendet, die beim Einsatz in einer PCR zur Amplifikation von Teilstücken der kodierenden Sequenz führen sollten. Mit einigen dieser Primerkombinationen konnten solche Teilstücke aus den in Beispiel 3 beschriebenen Stämmen isoliert werden. Für PCR-Reaktionen wurden durchweg kommerzielle Kits, in der Regel der PCR-Master-Kit der Fa. Roche, nach den Angaben der Herstellers verwendet. Mit den Primern
GDH-fwd P1 (5'-CCA GAT AAT CAA ATT TGG TTA AC-3') und

GDH-rev P7 (5'-CAT CAC GAT AAC GGT TYT TGC-3') konnten etwa 1200 bp große Fragmente isoliert werden. Anschließend wurden die erhaltenen DNA-Stücke sequenziert. Um die komplette Sequenz der einzelnen Gene zu erhalten, wurde eine Invers-PCR durchgeführt (Sambrook and Russell: Molecluar Cloning - A Laboratory Manual. CSHL Press (2001), p. 8.81). Im vorliegenden Fall wurden dazu Primer eingesetzt, die jeweils am Rand des Teilstückes so positioniert waren, dass in einer weiteren PCR der noch unbekannte Teil des GDH-Gens synthetisiert wird. Eine solche PCR-Reaktion wurde auf genomischer DNA des jeweiligen Stammes ausgeführt, die mit den Restriktionsendonukleasen EcoR1 bzw. BglII geschnitten und danach durch T4-Ligase rezirkularisiert worden war. Als Primer wurden
GDH-3Mid (5'-GGGATATGACCTACATTTGCTGGC-3') und

GDH-5Mid (5'-TGTCCATCAGCRTCATTTACAAYCTCAG-3') benutzt, die in Richtung des 3'-Endes (GDH-3Mid) bzw. 5'-Endes (GDH-5Mid) des GDH-Gens gerichtete DNA-Synthese initiierten. Die so erhaltenen Amplifikate enthielten die noch fehlenden Anteile der kodierenden Sequenz, wie sich durch Klonierung in den Vektor pCR2.1 und anschließende Sequenzierung herausstellte. Mit den nun komplett vorliegenden DNA-Sequenzen wurden erneut Primer entwickelt, die eine direkte Klonierung der kodierenden Sequenz vom Startcodon bis zum Stopcodon ermöglichten. Die Primer wurden dabei so gewählt, dass eine Klonierung in den Vektor pASK-IBA3 nach den Angaben der Fa. IBA möglich ist. Dazu wird eine BsaI-Schnittstelle unmittelbar vor der kodierenden Sequenz (im 5'-bindenden Primer) und eine unmittelbar danach (im 3'-bindenden Primer) so positioniert, dass eine gerichtete Ligation des mit BsaI geschnittenen PCR-Produkts in den mit BsaI geöffneten Vektor pASK-IBA3 möglich wird. Als 5'-Primer wurde GDH-U3(5'-TGGTAGGTCTCAAATGAATAAACATTTATTGGCTAAAATTAC-3'), als 3'-Primer wurden GDH-L3
(5'-ATGGTAGGTCTCAGCGCTCTGAGCTTTATATGTAAACCTAATCAAAG-3'; für die GDH aus Klon PT15) und GDH-L4
(5'-ATGGTAGGTCTCAGCGCTCTGAGCTTTATATGTAAATCTAATCAGAG-3';
für alle übrigen Klone) verwendet. Die entstandenen Plasmide wurden als pAI3-X bezeichnet. Anstelle von "X" wird die Stammbezeichnung des Klons eingesetzt, aus dem die genomische DNA isoliert wurde. Für Vergleichszwecke wurde in gleicher Weise das sPQQGDH-Gen aus dem Wildtypstamm LMD79.41 kloniert. Dazu wurden jedoch Primer verwendet die aus der publizierten Sequenz abgeleitet waren. Dieses Plasmid wurde mit pAI3-wt bezeichnet.

Die beschriebenen Plasmide wurden durch chemische Transformation in den E.coli-Stamm DH5α (Fa. Invitrogen) übertragen. Die so erhaltenen Bakterienstämme wurden als DH5α::pAI3-X bezeichnet. In ähnlicher Weise wurden die Plasmide in einen weiteren E.coli-Stamm, W3110 (ATCC 27325), übertragen. Diese Stämme wurden dann als W3110::pAI3-X bezeichnet.

### Beispiel 5: Rekombinante Herstellung von sPQQGDH mit E. coli

Die Vorkultur wurde wie folgt hergestellt. 2 ml LB-Medium (50 µg/ml Ampicillin) wurden mit 0,1 ml eines Glycerinstocks von DH5α::pAI3-KOZ65 Zellen versetzt und über Nacht bei 37°C und 225 rpm geschüttelt. Für die Hauptkultur wurden 1 L TB-Medium (50 µg/ml Ampicillin) mit der ausgewachsenen Vorkultur beimpft. Die Kultur wurde bei 37°C und 225 rpm geschüttelt, bis eine OD von ca. 1 erreicht war. Dann erfolgte die Induktion der Hauptkultur mit einer Anhydrotetracyclin (AHT) - Stammlösung. Der Induktor war dazu in DMF gelöst. Die Endkonzentration des AHT in der Kultur betrug 0,2 µg/ml, die Induktion erfolgte über 24 Stunden bei 27°C und 225 rpm. Die OD der Hauptkultur betrug dann 4,2.

Das Ernten der Zellen erfolgte durch Zentrifugation der Hauptkultur bei 3220 x g für 15 min bei 4°C. Die Zellpellets wurden in 40 ml (= 1/25 des Gesamtvolumens) in 75 mM Tris-HCl pH 8,0 resuspendiert und mittels French Press aufgeschlossen. Hierzu wird die gesamte Zellsuspension zweimal mit der French Press behandelt. Das evtl. durch Inclusion Bodies und Zelltrümmer getrübte Lysat wurde bei 48.745 x g für 10 min bei 4°C zentrifugiert. Der Proteingehalt und die Aktivität des Überstandes wurden getestet. Der Proteingehalt betrug 9,25 mg/ml, die Aktivität betrug 1,4 kU/ml. Bezogen auf die ursprüngliche Kultur wurden 56 kU pro L Kultur erhalten.

### Beispiel 6: Reinigung der rekombinanten sPQQGDH

11,2 ml des Überstand aus Beispiel 5 wurde über einen Kationenaustauscher (Toyopearl CM-650M, Fa. TOSOH BIOSEP GmbH) bei 4°C chromatographisch getrennt. Hierzu wird eine XK 50/20-Säule (Fa. Amersham Pharmacia Biotech) mit ca. 130 mL Säulenbett eingesetzt; die Flussrate betrug 8 mL/min. Die Säule wurde mit 10 Säulenvolumen 10 mM K-MOPS pH 8,0 + 1 mM CaCl₂ äquilibriert, danach erfolgt der Probenauftrag. Die Säule wurde mit 4 Säulenvolumen 10mM K-MOPS pH 8,0 + 1mM CaCl₂ gewaschen, anschließend wurde sie mittels eines linearen Salzgradienten von 0 bis 0,4 N NaCl in 10 mM K-MOPS pH 8,0 (jeweils incl. 1mM CaCl₂) eluiert, das Eluat wurde in Fraktionen aufgefangen. Die Regeneration der Säule erfolgt mit 3 Säulenvolumen 1 N NaCl + 1 mM CaCl₂,

In 96-well-Mikrotiterplatten mit Flachboden wurden die Eluat-Fraktionen auf Aktivität getestet, um die aktiven Fraktionen zu finden. Die Färbelösung war wie folgt zusammengesetzt:
0,2 mM PMS (Phenazine methosulfate in H₂O)
+ 0,22 mM NTB (Nitrotetrazolium blue in H₂O)
+ 3 µM PQQ-Na-Salz (in DMSO)
in 20mM Tris/HCl pH 7,5 mit 2 % Glucose

Der Test wurde wie folgt durchgeführt: Je 90 µL Probe wurden in einer Mikrotiterplatte vorgelegt und je 110 µL Farblösung wurden zugesetzt, die Farbreaktion kann meist bereits nach einer Minute beobachtet werden. Aufgrund der Ergebnisse im online-UV-Chromatogramm und im Aktivitätstest werden die aktiven Fraktionen vereint und der Pool ggf. durch Ultrafiltration (30.000 MWCO) aufkonzentriert. Es folgt eine Proteinbestimmung sowie ein quantitativer Aktivitätstest (s. Beispiel 9).

**Tabelle 2: Aufreinigung der sPQQGDH aus E. coli DH5α::pAI3-KOZ65**

| | **Vol. [mL]** | **Gesamt-protein [mg]** | **Gesamt-aktivität [U]** | **Spezif. Aktivität [U/mg]** | **Ausbeute [%]** |
|---|---|---|---|---|---|
| Probenauftrag auf Säule | 11,2 | 103,6 | 15680 | 151 | 100 |
| Aktive Fraktionen | 4,45 | 13,50 | 15085,5 | 1117 | 96 |

Im SDS Gel und im nativen Gel wurde nur eine aktive Bande detektiert. Das Protein war also sauber. Bezogen auf die urprüngliche Kultur wurden 48,2 mg reines Protein pro L Kultur erhalten.

### Beispiel 7: Herstellung rekombinanter sPQQGDH aus W3110-Stämmen und Prüfung der Thermostabilität

Für einen Vergleich der Thermostabilität von rekombinanter sPQQGDH aus LMD79.41 (Plasmid pAI3-wt) und der sPQQGDH aus KOZ65 (Plasmid pAI3-KOZ65) wurden die Stämme W3110::pAI3-wt und W3110::pAI3-KOZ65 in 200 ml TB-Medium mit 100 µg/ml Ampicillin angezogen. Nachdem die Kulturen eine OD₆₀₀ von 3 erreicht hatten, wurden die Bakterien bei 4.600 rpm für 10' zentrifugiert und die Pellets in je 25 ml frischem TB-Medium mit 100 µg/ml Ampicillin aufgenommen. Zur Induktion wurde AHT zu 2 µg/ml zugesetzt und die Zellen wurden bei 22°C für 6 h geschüttelt. Danach wurden die Zellen erneut pelletiert und die Pellets bis zur weiteren Verarbeitung bei -80°C gelagert. Die eingefrorenen Zellen wurden in je 25 ml MOPS-Puffer (10 mM MOPS pH 8, 2,5 mM CaCl₂, 0,05 % Triton X-100) resuspendiert und durch Ultraschallbehandlung aufgeschlossen, bis die Suspension deutlich klarer wurde. Zellreste wurden bei 20.000 rpm für 30 min bei 4°C abgetrennt und der Überstand über eine Toyopearl CM-650 M Säule (20 ml Bettvolumen) gereinigt. Dazu wurde die Säule nach dem Probenauftrag mit 50 ml MOPS-Puffer (s.o.) gewaschen und anschließend wurde gebundenes Protein mit einem Gradienten von 0 bis 0,6 mM NaCl in MOPS-Puffer eluiert. Fraktionen mit GDH-Aktivität wurden gepoolt und die Aktivität des Pools bestimmt (s. Beispiel 9).

Für die Bestimmung der Thermostabilität beider Enzympräparationen wurde durch Verdünnung mit 50 mM Pipes, pH 6.5 mit 1 mM CaCl₂, 0,1 % Triton X-100, 0,1 % BSA und 5 µM PQQ eine Lösung von 20 U/ml eingestellt. Von dieser Lösung wurden Aliquots parallel für 60 min bei 4°C, 50°C, 57°C und 64°C inkubiert. Danach wurden folgende Restaktivitäten, bezogen auf den Wert bei 4°C als 100 %, ermittelt (als Triplikate; s. Beispiel 9):

| **Stamm** | **50°C** | **57°C** | **64°C** |
|---|---|---|---|
| W3110::pAI3-wt | 95,1 ± 2,9 | 20,7 ± 0,8 | 3,4 ± 0,0 |
| W3110::pAI3-KOZ65 | 95,1 ± 1,6 | 56,8 ± 3,6 | 8,7 ± 0,3 |

### Beispiel 8: Herstellung mittels Hochzelldichtefermentation

Die Fermentation wurde in einem 10-Liter-Stahlfermenter (BIOSTAT C) der Firma Braun durchgeführt.

Es wurden dazu 5 Liter Medium nach Riesenberg (modifiziert) eingesetzt

| | | |
|---|---|---|
| KH₂PO₄ | | 13,3g |
| (NH₄)₂HPO₄ | | 4,0g |
| Zitronensäure | | 1,7g |
| Magnesiumsulfat x 7 H₂O | | 1,2g |
| Thiamin | | 0,5 g |
| Trypton | | 1,2 g |
| Hefeextrakt | | 2,4 g |
| Spurenelementlösung | | 50 ml |
| Aqua dest. | ad | 880ml |
| Glucose | | 5 g in 100 ml Aqua dest. (wird separat sterilisiert) |
| Ampicillin | | 100 mg (separat gelöst in 20 ml Aqua dest. und sterilfiltriert) |

### Spurenelementlösung

| | | |
|---|---|---|
| Titriplex III | | 0,84 g |
| Fe(III)citrat | | 6,00 g |
| MnCl2 x 4 H₂O | | 1,50 g |
| ZnCl2 x 2 H₂O | | 0,80 g |
| H₃B0₃ | | 0,30 g |
| Na₂MoO₄ x 2 H₂O | | 0,25 g |
| CoCl₂ x 6 H₂O | | 0,25 g |
| CuCl₂ x 2 H₂O | | 0,15 g |
| Aqua dest. | ad | 1000 ml |

Der pH wurde nach Zugabe aller Medienbestandteile mit 5 N NaOH auf 6,80 eingestellt.

Die Nährlösung wurde mit 100 ml einer über Nacht bei 37°C gewachsenen Vorkultur (LB-Medium mit 100 mg/L Ampicillin) beimpft.

Die OD nach der Beimpfung betrug 0,066. Die Belüftungsrate wurde auf 2 L/Min., die Rührerdrehzahl auf 500 UpM und die Temperatur auf 37°C eingestellt. Der pH-Wert wurde mit 5 N NH₃ auf pH 7,25 eingestellt und während der gesamten Fermentation konstant gehalten.

Der pO₂-Wert war nach 12 Stunden Wachstum auf unter 10 % gesunken und die Glucose zum größten Teil verbraucht. Nach 15 Stunden Wachstum betrug die OD₆₀₀ 14,2 und die Trockenmasse 6,2 g/L. Zu diesem Zeitpunkt wurde die Fütterung gestartet. Die Fütterlösung bestand aus:

| | | |
|---|---|---|
| Glukose | | 700 g |
| Magnesiumsulfat x 7 H₂O | | 28 g |
| Thiamin | | 0,5 g |
| Trypton | | 1,2 g |
| Hefeextrakt | | 2,4 g |
| Aqua dest. | ad | 1000 ml |

Die Lösung wurde sterilfiltriert. Sie wurde mittels einer Schlauchpumpe dem Fermenter zugeführt. Die Pumpleistung wurde über den pO₂-Sollwert geregelt, der auf 20 % eingestellt wurde. Die Regelung erfolgte so, dass bei einem pO₂-Wert >20 % die Pumpe eingeschaltet wird und neue Glucose zuführt. Der einsetzende Sauerstoffverbrauch lässt den pO₂ nun wieder sinken. Sinkt der pO₂-Wert unter 20 % wird die Pumpe abgeschaltet. Drehzahl und Belüftungsrate wurden nicht verändert. Nach 64 Stunden Wachstum betrug die OD₆₀₀ 50,2. Nun wurden dem Fermenter 5 Liter doppelt konzentriertes TB-Medium zugeführt, die Temperatur auf 22°C reduziert und die Belüftungsrate auf 4L /Min. eingestellt.

Die Rührerdrehzahl wurde auf 700 UpM erhöht. Dann wurde die Kultur mit 10 ml Anhydrotetracyclinlösung (2 mg/ml in DMF) 6 Stunden induziert. Anschließend wurde eine Probe zur Aktivitätsbestimmung entnommen. Der Aktivitätstest ergab einen Wert von 223 U / ml Kulturflüssigkeit. Über 220 mg reines Enzym wurden pro ml Kultur erhalten.

### Beispiel 9: Durchführung des Aktivitätstests:

Die Messungen wurden an einem Photometer "Ultraspec 2000" durchgeführt. Folgende Lösungen werden dafür eingesetzt:

| | |
|---|---|
| PIPES: | 50 mM pH 6,5 incl. 2,2 % Triton X-100 |
| Glucose: | 1 M in H₂O |
| PMS: | 3 mM Phenazine methosulfate in H₂O |
| NTB: | 6,6 mM Nitrotetrazolium blue in H₂O |
| CaCl₂: | 1 M in H₂O |
| PQQ: | 3 mM in DMSO |
| EDB *: | 50mM PIPES pH 6,5 incl. 0,1 % Triton X-100, 1mM CaCl₂, 0,1 % BSA, 6µM |
| | PQQ. |

EDB = Enzyme Dilution Buffer

Das Arbeitsreagenz besteht aus folgenden Komponenten:
25,5 ml PIPES incl. 2,2 % Triton X-100
0,9 ml Glucose
2,0 ml PMS
1,0 ml NTB

Der EDB und das Arbeitsreagenz sollten möglichst frisch angesetzt werden.

### Durchführung der Aktivitätsbestimmung:

20 µl der gewählten Proben-Verdünnung in Mikroküvetten vorlegen (als Nullwert werden 20 µl EDB anstelle der Probenlösung zugegeben),
600 µl Arbeitsreagenz zufügen und sofort die Messung bei 570 nm über 3 Minuten starten.

### Berechnung der Aktivität:

Die Absorptionsänderung wird als Extinktionsänderung/min ermittelt; eine Unit GDH erzeugt 0,5 µmol Formazan pro Minute. Es gilt folgende Formel:
U/ml = Extinktionsänderung/min x 1,54 x Verdünnungsfaktor ε = 40.200 M⁻¹cm⁻¹

## Patentansprüche

1. Lösliche Pyrrolochinolinchinon- bzw. Pyrroloqinolinquinon Glucose Dehydrogenase der Sequenz wie in Abbildung 1 dargestellt **dadurch gekennzeichnet, dass** folgende Aminosäuren gegenüber dem Wildtyp *Acinetobacter* LMD 79.41 ausgetauscht sind:
Position 21 ein N und 41 ein S und 47 ein L und 121 ein V oder ein A und 149 ein A und 213 ein S und 244 ein I und 320 ein G und 391 ein S und 452 ein S und 474 ein R und 480 ein Q als Aminosäure haben.

2. Lösliche Pyrrolochinolinchinon- bzw. Pyrroloqinolinquinon Glucose Dehydrogenase der Sequenzen aus Anspruch 1 **dadurch gekennzeichnet, dass** zusätzlich folgende Aminosäuren gegenüber dem Wildtyp *Acinetobacter* LMD 79.41 ausgetauscht sein können (Zählung gemäß Abbildung 1):
An der Positionen 16 kann ein H, an 18 ein L, an 20 ein F, an 40 ein G, an 48 ein I, an 61 ein A, an 111 ein T, an 154 ein D, an 190 ein E, an 293 ein A, an 311 ein S, an 314 ein A, an 324 ein L, an 333 ein M, an 339 ein S, an 355 ein G, an 366 ein D, an 417 ein N und an 418 ein A stehen.

3. Lösliche Pyrrolochinolinchinon- bzw. Pyrroloqinolinquinon Glucose Dehydrogenase der Sequenzen ensprechend der SEQ ID 5 bis SEQ ID 28.

4. Lösliche Pyrrolochinolinchinon- bzw. Pyrroloqinolinquinon Glucose Dehydrogenase der Sequenzen aus Anspruch 1 bis 3 **dadurch gekennzeichnet, dass** sie in den Vektor pASK-IBA3 kloniert sind und in einem Stamm der Gattung *Escherichia* heterolog exprimiert werden.

5. Verfahren zur Herstellung der löslichen Pyrrolochinolinchinon- bzw. Pyrroloqinolinquinon Glucose Dehydrogenase von *Acinetobacter* gemäß den Ansprüchen 1 bis 3 **dadurch gekennzeichnet, dass** man einen Mikroorganismus kultiviert der die lösliche Pyrrolochinolinchinon- bzw. Pyrroloqinolinquinonglucosedehydrogenase exprimiert und anschließend die lösliche Pyrrolochinolinchinon- bzw. Pyrroloqinolinquinon Glucose Dehydrogenase isoliert.

6. Reagenz zum Nachweis von Glucose enthaltend eine oder mehrere lösliche Pyrrolochinolinchinon- bzw. Pyrroloqinolinquinon Glucose Dehydrogenase Enzyme gemäß den Ansprüchen 1 bis 3.

7. Sensor zum Nachweis von Glucose enthaltend ein oder mehrere lösliche Pyrrolochinolinchinon- bzw. Pyrroloqinolinquinon Glucose Dehydrogenase Enzyme gemäß den Ansprüchen 1 bis 3.

8. Verwendung der mehrere lösliche Pyrrolochinolinchinon- bzw. Pyrroloqinolinquinon Glucose Dehydrogenase Enzyme gemäß den Ansprüchen 1 bis 3 zum Nachweis von Glucose.

## Claims

1. Soluble pyrroloquinoline-quinone glucose dehydrogenase of the sequence as depicted in Figure 1, **characterized in that** the following amino acids are exchanged by comparison with the wild type *Acinetobacter* LMD 79.41:
position 21 has an N and 41 has an S and 47 has an L and 121 has a V or an A and 149 has an A and 213 has an S and 244 has an I and 320 has a G and 391 has an S and 452 has an S and 474 has an R and 480 has a Q as amino acid.

2. Soluble pyrroloquinoline-quinone glucose dehydrogenase of the sequences from Claim 1, **characterized in that** additionally the following amino acids may be exchanged by comparison with the wild type *Acinetobacter* LMD 79.41 (numbering as shown in Figure 1).:
there may be at positions 16 an H, at 18 an L, at 20 an F, at 40 a G, at 48 an I, at 61 an A, at 111 a T, at 154 a D, at 190 an E, at 293 an A, at 311 an S, at 314 an A, at 324 an L, at 333 an M, at 339 an S, at 355 a G, at 366 a D, at 417 an N and at 418 an A.

3. Soluble pyrroloquinoline-quinone glucose dehydrogenase of the sequences according to SEQ ID 5 to SEQ ID 28.

4. Soluble pyrroloquinoline-quinone glucose dehydrogenase of the sequences from Claim 1 to 3, **characterized in that** they are cloned into the vector pASK-IBA3 and are heterologously expressed in a strain of the genus *Escherichia.*

5. Process for preparing the soluble pyrroloquinoline-quinone glucose dehydrogenase of *Acinetobacter* according to Claims 1 to 3, **characterized in that** a microorganism which expresses the soluble pyrroloquinoline-quinone glucose dehydrogenase is cultivated, and then the soluble pyrroloquinoline-quinone glucose dehydrogenase is isolated.

6. Reagent for detection of glucose comprising one or more soluble pyrroloquinoline-quinone glucose dehydrogenase enzymes according to Claims 1 to 3.

7. Sensor for detecting glucose comprising one or more soluble pyrroloquinoline-quinone glucose dehydrogenase enzymes according to Claims 1 to 3.

8. Use of the plurality of soluble pyrroloquinoline-quinone glucose dehydrogenase enzymes according to Claims 1 to 3 for detecting glucose.

## Revendications

1. Pyrroloquinoléinequinone-glucose-déshydrogénase soluble de séquence telle que représentée sur la figure 1, caactérisée en ce que les acides aminés suivants sont échangés par rapport à *Acinetobacter* LMD 79.41 de type sauvage :
les positions ont, comme acide aminé, 21 un N, 41 un S, 47 un L, 121 un V ou un A, 149 un A, 213 un S, 244 un I, 320 un G, 391 un S, 452 un S, 474 un R et 480 un Q.

2. Pyrroloquinoléine quinone-glucose-déshydrogénase soluble de séquences suivant la revendication 1, **caractérisée en ce que** les acides aminés suivants peuvent en outre être échangés par rapport à *Acinetobacter* LMD 79.41 de type sauvage (numérotage suivant figure 1):
il peut y avoir en positions : 16 un H, 18 un L, 20 un F, 40 un G, 48 un 1, 61 un A, 111 un T, 154 un D, 190 un E, 293 un A, 311 un S, 314 un A, 324 un L, 333 un M, 339 un S, 355 un G, 366 un D, 417 un N et 418 un A.

3. Pyrroloquinoléinequinone-glucose-déshydrogénase soluble de séquences correspondant à SEQ ID 5 jusqu'à SEQ ID 28.

4. Pyrroloquinoléinequinone-glucose-déshydrogénase soluble de séquences suivant les revendications 1 à 3, **caractérisée en ce qu'**elle est clonée dans le vecteur pASK-IBA3 et exprimée de façon hétérologue dans une souche du genre *Escherischia.*

5. Procédé de production de la pyrroloquinoléinequinone-glucose-déshydrogénase soluble d'*Acinetobacter* suivant les revendications 1 à 3, **caractérisé en qu'**on cultive un microorganisme qui exprime la pyrroloquinoléinequinone-glucose-déshydrogénase soluble et on isole ensuite la pyrroloquinoléinequinone-glucose-déshydrogénase soluble.

6. Réactif de détection de glucose, contenant comme enzymes une ou plusieurs pyrrologuinoléinequinone-glucose-déshydrogénases solubles suivant les revendications 1 à 3.

7. Détecteur de recherche du glucose, contenant comme enzymes une ou plusieurs pyrroloquinoléinequinone-glucose-déshydirogénases solubles suivant les revendications 1 à 3.

8. Utilisation des divers enzymes consistant en pyrroloquinoléinequinone-glucose-déshydrogénases solubles suivant les revendications 1 à 3 pour la détection de glucose.
